# EUROPEAN PATENT APPLICATION

(11) **EP 1 535 512 A1**
(43) Date of publication of application: **01.06.2005**
(21) Application number: 03764192.5
(22) Date of filing: 14.07.2003
(51) Int. Cl.: A01K 67/027, C12N 15/09, C12N 5/10, C12Q 1/02, C12Q 1/68, G01N 33/15, G01N 33/50, A61K 45/00, A61P 1/16, A61P 3/04, A61P 3/06, A61P 3/10, A61P 7/02, A61P 7/06, A61P 9/10, A61P 9/12, A61P 13/12, A61P 15/00, A61P 17/02, A61P 25/28, A61P 27/02

(54) **DISEASE MODEL ANIMAL CARRYING FOREIGN PPARa GENE TRANSFERRED THEREINTO AND USE THEREOF**

(30) Priority: 15.07.2002 JP 2002206162
(71) Applicant: Takeda Pharmaceutical Company Limited, Osaka-shi, Osaka 541-0045 (JP)
(72) Inventor: AMANO, Yuichiro, Osaka-shi, Osaka 533-0022 (JP); SUGIYAMA, Yasuo, Kawanishi-shi, Hyogo 666-0111 (JP); NISHIDA, Mayumi, Higashiosaka-shi, Osaka 579-8053 (JP); TAKETOMI, Shigehisa, Nishinomiya-shi, Hyogo 662-0095 (JP)
(74) Representative: Helbing, Jörg, Dr. Dipl.-Chem.
(86) International application number: PCT/JP2003/008921
(87) International publication number: WO 2004/006662

(57) **Abstract**

The present invention provides a non-human mammal, or a part of its living body, which stably retains a DNA encoding a heterologous PPARα in an expressible state, and has one or more different genetic modifications resulting in a pathological condition identical or similar to a disease associated with the regulation of PPARα activity or a foreign DNA under the control of a promoter having PPRE, as well as a method of screening for agonists/antagonists for the heterologous PPARα using the animal.

## Description

### Technical Field

The present invention relates to a heterologous PPARα gene-introduced non-human mammal. To be specific, the present invention relates to a non-human mammal into which a heterologous PPARα gene is introduced and which has a genetic modification showing a phenotype usable as an experimental animal model for a disease associated with the regulation of PPARα activity, a method of screening for an agent for the prophylaxis/treatment of various diseases associated with the regulation of PPARα activity, using the same, in the mammal from which the heterologous PPARα has been derived, and an agent for the prophylaxis/treatment of said diseases obtainable by said method.

### Background Art

Peroxisome Proliferator-Activated Receptor (hereinafter abbreviated as PPAR) α is a nuclear receptor type transcription factor that is highly expressed in liver, heart, kidney and the like, and plays a central role in lipid homeostasis through controlling the expression of genes encoding various proteins associated with lipid metabolism such as enzymes associated with β-oxidation of fatty acids and apolipoproteins. PPARα forms a heterodimer with retinoid X receptor (RXR), and binds, in the presence of its ligand, to Peroxisome Proliferator-Responsive Element (PPRE) sequence located in the 5' upstream of a target gene to promote the transcription of the gene. On the other had, it is known that PPARα also suppresses the expression of some genes in the presence of its ligand. It is thought to be due to the competition for the binding to a target DNA sequence or co-activator with other transcription factors.

PPARα is activated by a fat-soluble molecule such as a long-chain unsaturated fatty acid and a drug of fibrate class. A PPARα agonist enhances fatty acid oxidation activities in liver, on the other hand, it suppresses the expression of apolipoprotein C-III (apoC-III; a main constituent of plasma lipoproteins together with neutral fats (TG)). It is therefore effective to reduce the blood TG concentration. Since it promotes the expression of apolipoprotein A-I (apoA-I) and apolipoprotein A-II (apoA-II), which are main constituents of high density lipoprotein-cholesterols (HDL-C), it has a blood HDL-C concentration-increasing effect. Therefore, a PPARα agonist is actually used as an agent for the prophylaxis/treatment of hyperlipidemia including hypertriglyceridemia and hypo-HDL-cholesterolemia, and arteriosclerosis (for example, refer to Jean-Charles Fruchart et al., Current Atherosclerosis Reports, (USA), 2001, Vol. 3 (No. 1), p. 83-92) . A PPARα agonist is also known to show a heart-protective effect against ischemia (for example, refer to Antonia Tabernero et al., BMC Pharmacology,(UK), April 9, 2002 (published on-line), BioMed Central, internet <http://www.biomedcentral.com/1471-2210/2/10>).

A development of a novel PPARα agonist typically proceeds in the following order:
- carrying out *in vitro* screening such as a binding assay using human PPARα or expression assay of a gene under the control of PPRE (e.g., apoA-I, PPRE-reporter chimeric gene, etc.) in a human cell line, and
- evaluating *in vivo* effect of the compounds that has been confirmed to have a high activity by administration to an experimental animal such as mouse, rat, or the like.
However, due to the differences in PPARα structure between human and other mammals (for example, the amino acid identity between human and mouse is 92%), some drugs may show an agonistic activity only against human PPARα. Since such drugs show no activity against the endogenous PPARα of the experimental animal, they cannot be evaluated using existing animal models.

It is therefore an object of the present invention to provide a novel animal model allowing effective evaluation of the *in vivo* effect of a PPARα agonist showing an agonistic activity only against human PPARα, and a method of screening for an agent for the prophylaxis/treatment of various diseases such as hyperlipidemia, combined dyslipidemia, arteriosclerosis, hypertension, thrombosis, ischemic heart diseases, and ischemic brain diseases using said animal model.

### Disclosure of the Invention

The present inventors conducted extensive investigations with the aim of accomplishing the above-described object, and produced a transgenic mouse which stably retains a DNA encoding human PPARα in an expressible state. When a compound that has an activity against human PPARα but has no activity against mouse PPARα was administered to the mouse, it has been shown that said mouse showed various characteristic responses induced by a peroxisome proliferator including peroxisome proliferation and enhancement of the expression of lipid metabolism-related genes. Furthermore, the present inventors succeeded in producing human PPARα expressing animal models for various diseases associated with the control of PPARα activity including hyperlipidemia and arteriosclerosis by crossing the obtained human PPARα expressing transgenic mouse and experimental animal models for said diseases.

The present inventors conducted further investigations based on these findings, which resulted in the completion of the present invention.

Accordingly, the present invention provides:
[1] a non-human mammal, which stably retains a DNA encoding a heterologous PPARα in an expressible state and has one or more different genetic modifications, or a part of its living body;
[2] the animal or the part of its living body of the above-mentioned [1], wherein at least one of said different genetic modifications results in pathological condition(s) equal or similar to disease(s) associated with the regulation of PPARα activity;
[3] the animal or the part of its living body of the above-mentioned [1], wherein at least one of said different genetic modifications is introduction of a foreign DNA under the control of a promoter having PPRE;
[4] the animal or the part of its living body of the above-mentioned [1], wherein said heterologous PPARα is human derived PPARα;
[5] the animal or the part of its living body of the above-mentioned [1], wherein said heterologous PPARα has the same or substantially the same amino acid sequence as the amino acid sequence represented by SEQ ID NO: 2;
[6] the animal or the part of its living body of the above-mentioned [1], wherein said non-human mammal is rabbit, dog, cat, guinea pig, hamster, mouse or rat;
[7] the animal or the part of its living body of the above-mentioned [1], wherein said non-human mammal is mouse;
[8] the animal or the part of its living body of the above-mentioned [1], wherein said animal expresses said heterologous PPARα in place of lacking its endogenous PPARα;
[9] the animal or the part of its living body of the above-mentioned [8], wherein said animal is obtainable by crossing an endogenous PPARα-deficient animal and the same species of animal that expresses a heterologous PPARα;
[10] the animal or the part of its living body of the above-mentioned [8], wherein said endogenous PPARα is mouse-derived PPARα and said heterologous PPARα is human-derived PPARα;
[11] the animal or the part of its living body of the above-mentioned [2], wherein said diseases associated with the regulation of PPARα activity are one or more diseases selected from the group consisting of hyperlipidemia, hypertriglyceridemia, combined dyslipidemia, hypo-HDL-cholesterolemia, arteriosclerosis, peripheral arterial obstruction, intermittent claudication, gangrene, hypertension, thrombosis, ischemic heart disease, acute myocardial infarction, heart failure, congestive heart failure, unstable angina pectoris, post-PTCA restenosis, post-stenting restenosis, hyperfibrinogemia, cardiomyopathy, cerebral hemorrhage, transient ischemic attack, cerebral infarction, cerebral apoplexy, chronic glomerulonephritis, diabetic nephropathy, renal arteriosclerosis, dermatitis, immunodeficiency, hypoglycemia, hypoketonemia, fatty liver, diabetes mellitus, diabetic neuropathy, diabetic retinopathy, obesity, Alzheimer's disease, anemic hypoxia, gonadal dysfunction, liver cancer, breast cancer and endometritis;
[12] the animal or the part of its living body of the above-mentioned [1], wherein said heterologous PPARα is specifically expressed in one or more region selected from the group consisting of liver, heart, kidney, adrenal gland, blood vessel, gastrointestinal tract and brain;
[13] the animal or the part of its living body of the above-mentioned [1], wherein said heterologous PPARα is specifically expressed in liver;
[14] a method of screening for an agonist or antagonist for a heterologous PPARα, which comprises applying a test substance to the animal or the part of its living body of the above-mentioned [1], and assaying its agonistic or antagonistic activity against the heterologous PPARα;
[15] a method of screening for an agonist or antagonist for a heterologous PPARα, which comprises applying a test substance to the animal or the part of its living body of the above-mentioned [3], and assaying its agonistic or antagonistic activity against the heterologous PPARα using the expression of a foreign DNA under the control of a promoter having PPRE as an index; and
[16] a method of screening for a substance having a prophylactic/therapeutic activity for disease(s) associated with the regulation of PPARα activity in an animal from which a heterologous PPARα is derived, which comprises administering a test substance to the animal of the above-mentioned [2], and assaying effect(s) of the substance on pathological condition(s) equal or similar to disease(s) associated with the regulation of PPARα activity in the animal.
   Furthermore, the present invention provides:
[17] an agonist for a heterologous PPARα obtainable by the method of the above-mentioned [14] or [15];
[18] an agent for the prophylaxis/treatment of one or more diseases selected from the group consisting of hyperlipidemia, hypertriglyceridemia, combined dyslipidemia, hypo-HDL-cholesterolemia, arteriosclerosis, peripheral arterial obstruction, intermittent claudication, gangrene, hypertension, thrombosis, ischemic heart disease, acute myocardial infarction, heart failure, congestive heart failure, unstable angina pectoris, post-PTCA restenosis, post-stent restenosis, hyperfibrinogemia, cardiomyopathy, cerebral hemorrhage, transient ischemic attack, cerebral infarction, cerebral apoplexy, chronic glomerulonephritis, diabetic nephropathy, renal arteriosclerosis, dermatitis, immunodeficiency, hypoglycemia, hypoketonemia, fatty liver, diabetes mellitus, diabetic neuropathy, diabetic retinopathy, obesity, Alzheimer's disease, anemic hypoxia and gonadal dysfunction in an animal from which a heterologous PPARα is derived, which comprises the agonist of the above-mentioned [17];
[19] the agent for the prophylaxis/treatment of the above-mentioned [18], wherein said animal from which the heterologous PPARα is derived is human;
[20] a method for the prophylaxis/treatment of one or more diseases selected from the group consisting of hyperlipidemia, hypertriglyceridemia, combined dyslipidemia, hypo-HDL-cholesterolemia, arteriosclerosis, peripheral arterial obstruction, intermittent claudication, gangrene, hypertension, thrombosis, ischemic heart disease, acute myocardial infarction, heart failure, congestive heart failure, unstable angina pectoris, post-PTCA restenosis, post-stent restenosis, hyperfibrinogemia, cardiomyopathy, cerebral hemorrhage, transient ischemic attack, cerebral infarction, cerebral apoplexy, chronic glomerulonephritis, diabetic nephropathy, renal arteriosclerosis, dermatitis, immunodeficiency, hypoglycemia, hypoketonemia, fatty liver, diabetes mellitus, diabetic neuropathy, diabetic retinopathy, obesity, Alzheimer's disease, anemic hypoxia and gonadal dysfunction in an animal from which a heterologous PPARα is derived, which comprises administering to said animal an effective amount of the agonist of the above-mentioned [17];
[21] a use of the agonist of the above-mentioned [17] for the production of an agent for the prophylaxis/treatment of one or more diseases selected from the group consisting of hyperlipidemia, hypertriglyceridemia, combined dyslipidemia, hypo-HDL-cholesterolemia, arteriosclerosis, peripheral arterial obstruction, intermittent claudication, gangrene, hypertension, thrombosis, ischemic heart disease, acute myocardial infarction, heart failure, congestive heart failure, unstable angina pectoris, post-PTCA restenosis, post-stent restenosis, hyperfibrinogemia, cardiomyopathy, cerebral hemorrhage, transient ischemic attack, cerebral infarction, cerebral apoplexy, chronic glomerulonephritis, diabetic nephropathy, renal arteriosclerosis, dermatitis, immunodeficiency, hypoglycemia, hypoketonemia, fatty liver, diabetes mellitus, diabetic neuropathy, diabetic retinopathy, obesity, Alzheimer's disease, anemic hypoxia and gonadal dysfunction in an animal from which a heterologous PPARα is derived;
[22] an antagonist for a heterologous PPARα obtainable by the method of the above-mentioned [14] or [15];
[23] an agent for the prophylaxis/treatment of one or more diseases selected from the group consisting of liver cancer, breast cancer and endometritis in an animal from which a heterologous PPARα is derived, which comprises the antagonist of the above-mentioned [22];
[24] the agent for the prophylaxis/treatment of the above-mentioned [23], wherein said animal from which the heterologous PPARα is derived is human;
[25] the method for the prophylaxis/treatment of one or more diseases selected from the group consisting of liver cancer, breast cancer and endometritis in an animal from which a heterologous PPARα is derived, which comprises administering to said animal an effective amount of the antagonist of the above-mentioned [22];
[26] a use of the antagonist of the above-mentioned [22] for the production of an agent for the prophylaxis/treatment of one or more diseases selected from the group consisting of liver cancer, breast cancer and endometritis in an animal from which a heterologous PPARα is derived;
[27] a substance having a prophylactic/therapeutic activity for disease(s) associated with the control of PPARα activity in an animal from which a heterologous PPARα is derived, which is obtainable by the method of the above-mentioned [16];
[28] an agent for the prophylaxis/treatment of disease(s) associated with the control of PPARα activity in an animal from which a heterologous PPARα is derived, which comprises the substance of the above-mentioned [27];
[29] the agent for the prophylaxis/treatment of the above-mentioned [28], wherein said diseases associated with the control of the heterologous PPARα activity are one or more diseases selected from the group consisting of hyperlipidemia, hypertriglyceridemia, combined dyslipidemia, hypo-HDL-cholesterolemia, arteriosclerosis, peripheral arterial obstruction, intermittent claudication, gangrene, hypertension, thrombosis, ischemic heart disease, acute myocardial infarction, heart failure, congestive heart failure, unstable angina pectoris, post-PTCA restenosis, post-stent restenosis, hyperfibrinogemia, cardiomyopathy, cerebral hemorrhage, transient ischemic attack, cerebral infarction, cerebral apoplexy, chronic glomerulonephritis, diabetic nephropathy, renal arteriosclerosis, dermatitis, immunodeficiency, hypoglycemia, hypoketonemia, fatty liver, diabetes mellitus, diabetic neuropathy, diabetic retinopathy, obesity, Alzheimer's disease, anemic hypoxia and gonadal dysfunction;
[30] a method for the prophylaxis/treatment of disease(s) associated with the control of PPARα activity in an animal from which a heterologous PPARα is derived, which comprises administering to said animal an effective amount of the substance of the above-mentioned [27]; and
[31] a use of the substance of the above-mentioned [27] for the production of an agent for the prophylaxis/treatment of disease(s) associated with the control of PPARα activity in an animal from which a heterologous PPARα is derived, and the like.

### Brief Description of the Drawing

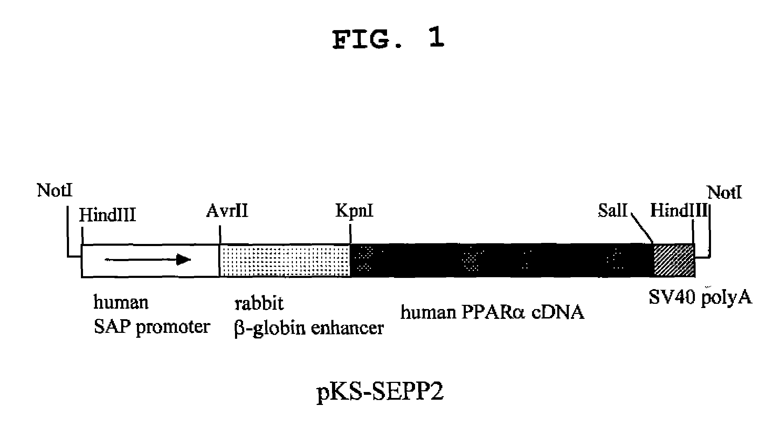

Fig. 1 is a drawing showing a restriction enzyme map of a human PPARα expression vector pKS-SEPP2. The arrow in the figure shows the direction of transcription.

### Best Mode for Carrying Out the Invention

The heterologous PPARα gene-introduced non-human mammal of the present invention is an animal model allowing evaluation of the efficacy of a PPARα agonist or antagonist, *in vivo,* which acts on the heterologous PPARα, but not on the endogenous PPARα of the non-human mammal.

The non-human mammal stably retaining a DNA encoding a heterologous PPARα in an expressible state (hereinafter, also referred to as "the transgenic non-human mammal of the present invention" or simply "transgenic animal of the present invention") "stably retains" a DNA encoding a heterologous PPARα in an expressible state. "stably retains" means that the DNA encoding the heterologous PPARα durably exists in an expressible state within the cells of said animal. Although said DNA may be integrated into the host chromosome or stably exist as an extrachromosomal DNA, it is preferably retained in the state of being integrated into the host chromosome.

The transgenic animal of the present invention can be prepared by transfecting a DNA encoding the heterologous PPARα of interest into a fertilized egg, unfertilized egg, spermatozoon or precursor cell thereof (primordial germ cell, oogonium, oocyte, egg cell, spermatogonium, spermatocyte, sperm cell or the like), preferably in the early stage in the embryogenic development (more preferably before the 8-cell phase) of the non-human mammal, by a gene transfer method, such as the calcium phosphate coprecipitation method, the electroporation method, the lipofection method, the agglutination method, the microinjection method, the particle gun method, the DEAE-dextran method, etc. Also, it is possible to transfect the DNA of interest into a somatic cell, tissue, organ or the like of the non-human mammal by the gene transfer method, and utilize the transformant for cell culture, tissue culture, etc. In addition, these cells may be fused with the above-described embryo (or germ) cell by a publicly known cell fusion method to produce the transgenic animal. Alternatively, the transgenic animal can also be obtained, in the same manner as producing a knockout animal, by transfecting the DNA of interest into embryonic stem cells (ES cells) of the non-human mammal using the above-described gene transfer method, selecting clones stably incorporating the DNA, injecting the ES cells into a blastocyst or aggregating an ES cell mass with 8-cell embryos to produce chimeric mice, and selecting one in which the introduced DNA has been transmitted to germ line.

A part of the living body of the transgenic animal produced in this manner (for example, (1) a cell, tissue, organ or the like stably retaining a DNA encoding a heterologous PPARα; (2) ones obtained by culturing and, if necessary, subculturing a cell or tissue derived therefrom, etc.) can be used for the same purpose as "the non-human mammal stably retaining a DNA encoding a heterologous PPARα in an expressible state" of the present invention, as "a part of the living body of the non-human mammal stably retaining a DNA encoding a heterologous PPARα in an expressible state" of the present invention.

As examples of the part of the living body of the transgenic animal of the present invention, organs such as the liver, heart, kidney, adrenal gland, blood vessels, gastrointestinal tract and brain, and tissue sections and cells derived from these organs, and the like can be mentioned.

The "non-human mammal" that can be used as the subject of the present invention is not subject to limitation, as long as it is a non-human mammal for which a transgenic system has been established; for example, bovine, monkey, swine, sheep, goat, rabbit, dog, cat, guinea pig, hamster, rat, mouse and the like can be mentioned. Preferably, rabbit, dog, cat, guinea pig, hamster, mouse, rat and the like can be mentioned; from the viewpoint of preparation of a disease model animal, in particular, rodent animals, which have relatively short ontogeny and biological cycles, and which permit easy propagation, are more preferable, and mice (for example, C57BL/6 strain, DBA2 strain and the like as pure strains, B6C3F₁ strain, BDF₁ strain, B6D2F₁ strain, BALB/c strain, ICR strain and the like as crossed strains) and rats (for example, Wistar, SD and the like) are particularly preferable.

Also, in addition to mammals, birds such as chicken can be used for the same purpose as that of a "non-human mammal" that is the subject of the present invention.

"A DNA that encodes a heterologous PPARα" means a DNA that encodes a PPARα derived from a mammal heterologous to the non-human mammal (for example, mouse) to be the subject of gene transfer (for example, human, bovine, monkey, swine, sheep, goat, rabbit, dog, cat, guinea pig, hamster, rat and the like), or a protein having substantially the same amino acid sequence as the PPARα. Preferably, the DNA that encodes a heterologous PPARα of the present invention is a DNA that encodes the human PPARα or a protein having substantially the same amino acid sequence as the human PPARα. As the DNA that encodes the human PPARα, a DNA that encodes the amino acid sequence shown by SEQ ID NO: 2, preferably a DNA that has the base sequence shown by SEQ ID NO: 1, can be mentioned. As examples of "substantially the same amino acid sequence", in the case of the human PPARα, an amino acid sequence possessing an identity of about 90% or higher, preferably 95% or higher, more preferably about 98% or higher, to the amino acid sequence shown by SEQ ID NO: 2, and the like can be mentioned. Amino acid sequence identity can be calculated using the homology calculation algorithm NCBI BLAST (National Center for Biotechnology Information Basic Local Alignment Search Tool) under the following conditions (expectancy=10; gap allowed; matrix=BLOSUM62; filtering=OFF).

As examples of the "protein having substantially the same amino acid sequence", in the case of the human PPARα, a protein having substantially the same amino acid sequence as the amino acid sequence shown by SEQ ID NO: 2, and possessing substantially the same quality of activity as a protein having the amino acid sequence shown by SEQ ID NO: 2, is preferred. As examples of "substantially the same quality of activity", ligand (including agonist and antagonist) binding activity, regulatory action on lipid metabolism-related gene expression and the like can be mentioned. "Substantially the same quality of activity" indicates that their activities are qualitatively equivalent to each other. Therefore, it is preferable that activities such as ligand-binding activity and regulatory action on lipid metabolism-related gene expression be equivalent (e.g., about 0.01 - 100 folds, preferably about 0.5 - 20 folds, more preferably about 0.5 - 2 folds), but quantitative factors such as the degrees of these activities and protein molecular weight may differ. Measurements of activities such as ligand-binding activity and regulatory action on lipid metabolism-related gene expression can be conducted in accordance with a method known per se, and can be measured according to, for example, the screening method described below.

Although the DNA that encodes a heterologous PPARα is preferably in an intron-free state (that is, a complementary DNA) like, for example, a DNA having the base sequence shown by SEQ ID NO: 1, an intron-containing form (that is, genomic DNA) can also be used preferably in another mode of embodiment because the 5' and 3' terminal sequences of the intron are common to almost all eukaryotic organism genes.

The DNA that encodes a heterologous PPARα can be isolated by the hybridization method or the PCR method and the like, with an oligonucleotide prepared on the basis of a known PPARα gene sequence as the probe or primer, using a DNA derived from the liver, heart, kidney, adrenal gland, blood vessel, gastrointestinal tract and the like of a human or one of various non-human mammals (bovine, monkey, swine, sheep, goat, rabbit, dog, cat, guinea pig, hamster, rat, mouse and the like) and all or a portion of a genomic DNA derived from one of various commercially available genomic DNA libraries as the raw material, or using a cDNA prepared from an RNA derived from the liver, heart, kidney, adrenal gland, blood vessel, gastrointestinal tract and the like of a human or one of various non-human mammals by a known method as the raw material.

The transgenic animal of the present invention retains a DNA that encodes a heterologous PPARα in "an expressible state". Therefore, in introducing the DNA to the subject animal, it is generally advantageous to use the DNA in a form containing an expression cassette joined downstream of a promoter capable of functioning in the cells of the subject animal (e.g., expression vector and the like).

As the vector that harbors a DNA that encodes a heterologous PPARα, an Escherichia coli-derived plasmid, a Bacillus subtilis-derived plasmid, a yeast-derived plasmid, a bacteriophage such as λ phage, a retrovirus such as Molony leukemia virus, an animal or insect virus such as vaccinia virus or baculovirus, and the like are used. Particularly, an Escherichia coli-derived plasmid, a Bacillus subtilis-derived plasmid or a yeast-derived plasmid and the like are preferably used, and an Escherichia coli-derived plasmid is especially preferable.

As examples of the promoter for regulation of the gene expression of a heterologous PPARα, promoters of genes derived from viruses (cytomegalovirus, Moloney leukemia virus, JC virus, breast cancer virus and the like), promoters of genes derived from various mammals (human, bovine, monkey, swine, sheep, goat, rabbit, dog, cat, guinea pig, hamster, rat, mouse and the like) and birds (chicken and the like) [for example, albumin, endothelin, osteocalcin, muscular creatine kinase, collagen type I and type II, cyclic AMP-dependent protein kinase βI subunit, atrial natriuretic factor, dopamine β-hydroxygenase, neurofilament light chain, metallothionein I and IIA, metalloproteinase 1 tissue inhibitor, smooth muscle α actin, polypeptide chain elongation factor 1α (EF1-α), β actin, α and β myosin heavy chains, myosin light chains 1 and 2, myelin basic protein, serum amyloid P component, renin and the like], and the like can be mentioned. Preferably, a promoter capable of expressing a heterologous PPARα specifically or at a high level in the target tissue according to the desired disease model (e.g., gene promoters expressible at high levels in the liver, such as serum amyloid P component (SAP), albumin, transferring fibrinogen, antithrombin III, α1-antitrypsin; gene promoters expressible at high levels in the heart, such as α and β myosin heavy chains and myosin light chains 1 and 2; gene promoters expressible at high levels in the kidney, such as PTH/PTHrP receptor; gene promoters expressible at high levels in the adrenal gland, such as ACTH receptor; gene promoters expressible at high levels in the gastrointestinal tract, such as fatty acid-binding proteins; gene promoters expressible at high levels in the brain, such as myelin basic protein and glial fibrillary acidic protein, and the like) can be appropriately selected. For example, when the transgenic animal of the present invention is a model of hyperlipidemia or arteriosclerosis, it is preferable to use a promoter expressible at a high level in the liver.

Downstream of the DNA that encodes a heterologous PPARα, a sequence that terminates the transcription of the desired messenger RNA (polyadenylation (polyA) signal, also referred to as terminator) in the transgenic animal is preferably present; for example, using a terminator sequence derived from a virus gene, or derived from a gene of one of various mammals or birds, it is possible to achieve efficient expression of the transferred gene. Preferably, the simian virus SV40 terminator and the like are used. In addition, to express the desired gene at a still higher level, the splicing signal of each gene, an enhancer region, or a portion of the intron of an eukaryotic gene can be joined upstream of the 5' of the promoter region, between the promoter region and the translational region, or downstream of the 3' of the translational region, depending on the purpose.

Also, when a transgenic animal is prepared using an embryonic stem cell (ES cell), the above-described vector preferably further contains a selection marker gene for selection of a clone having the transferred DNA incorporated stably therein (e.g., drug resistance genes such as the neomycin resistance gene and the hygromycin resistance gene). Furthermore, when it is intended to incorporate the transferred DNA in a particular site of a host chromosome by homologous recombination (that is, preparation of a knock-in animal), the above-described vector preferably further contains, to eliminate random insertions, the herpes simplex virus-derived thymidine kinase (HSV-tk) gene or the diphtheria toxin gene, as the negative selection marker gene, outside a DNA sequence homologous to the target site. These modes of embodiment are described in detail below.

The above-described promoter, a DNA that encodes a heterologous PPARα, a terminator and the like can be inserted to the above-described vector in the correct arrangement, that is, in an arrangement that enables the expression of the heterologous PPARα in the transgenic animal, by ordinary gene engineering techniques using appropriate restriction enzyme and DNA ligase and the like.

In another preferred mode of embodiment, the expression vector containing a DNA that encodes a heterologous PPARα obtained as described above, is transferred to an early embryo of the subject non-human mammal by the microinjection method.

An early embryo of the subject non-human mammal can be obtained by collecting an internally fertilized egg obtained by mating a female and a male of the same species of non-human mammal, or by externally fertilizing an ovum and sperm collected from a female and a male, respectively, of the same species of non-human mammal.

The age, breeding conditions and the like for the non-human mammal used vary depending on the animal species, for example; when using the mouse (preferably an inbred mouse such as C57BL/6J (B6), F₁ between B6 and another inbred line, and the like), it is preferable that the female be at about 4 to about 6 weeks of age, and the male be at about 2 to about 8 months or so of age, and is also preferable that they be reared under the conditions of about 12-hour light period (for example, 7:00-19:00) for about 1 week.

Although internal fertilization may be by spontaneous mating, a method wherein for the purpose of regulating the sexual cycle and obtaining a large number of early embryos from one animal, gonadotropine is administered to a female non-human mammal to induce over-ovulation, and thereafter the female is mated with a male non-human mammal, is preferred. As examples of the method of inducing ovulation in a female non-human mammal, a method wherein follicle-stimulating hormone (pregnant mare's serum gonadotropine, generally abbreviated as PMSG) is first administered, then luteinizing hormone (human chorionic gonadotropine, generally abbreviated as hCG) is administered, by, for example, intraperitoneal injection and the like, is preferred; the preferable hormone dosage and administration interval respectively vary depending on the species of non-human mammal. For example, when the non-human mammal is the mouse (preferably an inbred mouse such as C57BL/6J (B6), F₁ between B6 and another inbred line, and the like), a method wherein a fertilized egg is obtained by administering luteinizing hormone at about 48 hours after administration of follicle-stimulating hormone, and thereafter immediately mating the female with a male mouse, is usually preferred; the dosage of follicle-stimulating hormone is about 20 - about 50 IU/animal, preferably about 30 IU/animal, and the dosage of luteinizing hormone is about 0 - about 10 IU/animal, preferably about 5 IU/animal.

After a given time has elapsed, the abdominal cavity of each female non-human mammal confirmed by vaginal plug testing and the like to have copulated was opened, and fertilized eggs are taken out from the oviduct, washed in a medium for embryo culture (e.g., M16 medium, modified Whitten medium, BWW medium, M2 medium, WM-HEPES medium, BWW-HEPES medium and the like) to remove cumulus cells, and cultured by the droplet culture method and the like in the presence of 5% carbon dioxide gas/95% atmosphere until the time of DNA microinjection. When microinjection is not immediately conducted, it is also possible to preserve the collected fertilized eggs under freezing by the slow method or the ultrarapid method and the like.

On the other hand, in the case of external fertilization, follicle-stimulating hormone and luteinizing hormone are administered to a female non-human mammal for egg collection (the same as in the case of internal fertilization used preferably) in the same manner as above to induce ovulation, after which eggs are collected and cultured in a medium for fertilization (e.g., TYH medium) until the time of external fertilization by the droplet culture method and the like in the presence of 5% carbon dioxide gas/95% atmosphere. On the other hand, the tail of the epididymis is taken out from the same species of male non-human mammal (the same as in the case of internal fertilization is preferably used), and a sperm mass is collected and pre-cultured in a medium for fertilization. After completion of the pre-culture, the sperm is added to an egg-containing medium for fertilization; after cultivation by the droplet culture method and the like in the presence of 5% carbon dioxide gas/95% atmosphere, fertilized eggs having two pronucleuses are selected under a microscope. When DNA microinjection is not immediately conducted, it is also possible to preserve the collected fertilized eggs under freezing by the slow method or the ultrarapid method and the like.

DNA microinjection to a fertilized egg can be performed using a known apparatus such as a micromanipulator according to a conventional method. Briefly speaking, the fertilized egg placed in a droplet of a medium for embryo culture is aspirated and immobilized using a holding pipette, and a DNA solution is injected directly to the male or female pronucleus, preferably into the male pronucleus, using an injection pipette. The transferred DNA used is preferably one that has been highly purified by CsCl density ultracentrifugation and the like. Also, the transferred DNA is preferably linearized by cutting the vector portion thereof using a restriction endonuclease.

After the DNA transfer, the fertilized egg is cultured in a medium for embryo culture by the droplet culture method and the like in the presence of 5% carbon dioxide gas/95% atmosphere until the 1-cell stage - blastcyst stage, after which it is transplanted into the oviduct or uterus of a female non-human mammal for embryo reception rendered to be pseudopregnant. The female non-human mammal for embryo reception may be any female, as long as it is of the same species as the animal from which the early embryo to be transplanted is derived; for example, when a mouse early embryo is transplanted, a female ICR strain mouse (preferably about 8 to about 10 weeks of age) and the like are preferably used. As an example of the method of rendering the female non-human mammal for embryo reception to be in a pseudopregnant state, a method wherein the female is mated with the same species of vasectomized (vasoligated) male non-human mammal (for example, in the case of a mouse, a male ICR strain mouse (preferably about 2 months or more of age)), and selecting one confirmed as having a vaginal plug, is known.

The female for embryo reception used may be a spontaneously ovulating female, or a female having fertility induced by administering luteinizing hormone-releasing hormone (generally abbreviated as LHRH) or an analog thereof prior to mating with a vasectomized (vasoligated) male. As examples of the LHRH analog, [3,5-DiI-Tyr⁵]-LH-RH, [Gln⁸]-LH-RH, [D-Ala⁶]-LH-RH, [des-Gly¹⁰]-LH-RH, [D-His (Bzl)⁶] -LH-RH, Ethylamides thereof and the like can be mentioned. The dosage of LHRH or an analog thereof, and the timing of mating with a male non-human mammal after administration thereof vary depending on the species of non-human mammal. For example, when the non-human mammal is the mouse (preferably an ICR strain mouse and the like), it is usually preferable that the female mouse be mated with a male mouse at about 4 days after LHRH or an analog thereof is administered; the dosage of LHRH or an analog thereof is usually about 10 - 60 µg/animal, preferably about 40 µg/animal.

Usually, when the early embryo to be transplanted is in the morula stage or after, it is transplanted to the uterus of a female for embryo reception; when the early embryo is in an earlier stage (for example, 1-cell stage to 8-cell stage embryo), it is transplanted to the oviduct. As the female for embryo reception, one which is older than a given number of days from pseudopregnancy, depending on the developmental stage of the transplanted embryo, is appropriately used. For example, in the case of the mouse, a female mouse at about 0.5 days after pseudopregnancy is preferred for transplantation of a 2-cell stage embryo, and a female mouse at about 2.5 days after pseudopregnancy is preferred for transplantation of a blastcystic embryo. After the female for embryo reception is anesthetized (preferably Avertin, Nembutal and the like are used), an incision is made, the ovary is drawn out, early embryo (about 5 to about 10 cells) in suspension in a medium for embryo culture are injected to the peritoneal orifice of the oviduct or the vicinity of the oviduct junction of the uterine horn using a pipette for embryo transplantation.

If the transplanted embryo successfully implants and the embryo recipient female becomes pregnant, non-human mammal pups are obtained by spontaneous delivery or caesarian section. Embryo recipient females that delivered spontaneously are allowed to continue suckling; if the pups are delivered by caesarian section, the pups can be suckled by a separately provided female for suckling (for example, in the case of the mouse, a female mouse with usual mating and delivery (preferably female ICR strain mouse and the like)).

Referring to the introduction of a DNA that encodes a heterologous PPARα in the fertilized egg cell stage, it is assured that the transferred DNA is present in all germ line cells and somatic cells of the subject non-human mammal. Whether or not the transferred DNA is incorporated in the chromosome DNA can, for example, be determined by screening chromosome DNAs separated and extracted from the tails of offspring pups, by Southern hybridization or PCR method. The presence of a DNA that encodes a heterologous PPARα in the germ line cells of non-human mammal pups (F₀) obtained as described above means that a DNA that encodes a heterologous PPARα is present in all of the germ line cells and somatic cells of all progeny (F₁) animals.

Usually, the F₀ animals are obtained as heterozygotes having the transferred DNA in only one of the homologous chromosomes. Also, individual F₀ animals are randomly inserted onto different chromosomes unless produced by homologous recombination. To obtain a homozygote having a DNA that encodes a heterologous PPARα on both of the homologous chromosomes, an F₀ animal and a non-transgenic animal are crossed to prepare F₁ animals, and siblings of a heterozygote having the transferred DNA in only one of the homologous chromosomes are crossed to each other. Provided that the transferred DNA has been incorporated in only one gene locus, 1/4 of the obtained F₂ animals would be homozygotes.

In another preferred mode of embodiment, the expression vector containing a DNA that encodes a heterologous PPARα is transferred to an ES cell of the subject non-human mammal by a known method of gene introduction such as electroporation method.

An ES cell refers to a cell which is derived from the inner cell mass (ICM) of a fertilized egg in the blastcyst stage, and which can be cultured and maintained while retaining an undifferentiated state in vitro. Cells in the ICM are cells that will form the embryo itself and are also stem cells on which all tissues, including germ cells, are based. The ES cell may be of an already established cell line, and may also be newly established in accordance with the method of Evans and Kaufman (Nature, Vol. 292, p. 154, 1981). For example, in the case of a mouse ES cell, currently, an ES cell derived from the 129 strain mouse is generally used; however, since the immunological background is unclear, for example, an ES cell established from the C57BL/6 mouse or the BDF₁ mouse (F₁ between C57BL/6 and DBA/2), which has been developed by improving the low number of eggs collectable from C57BL/6 by crossing with DBA/2, and the like can also be used favorably for the purpose of instead obtaining an ES cell which is of a pure strain, and which has an immunologically clear genetic background and the like. Because the BDF₁ mouse has the C57BL/6 mouse as the background, in addition to being advantageous in that the number of collectable eggs is large and the eggs are tough, ES cells derived therefrom are advantageously usable in that the genetic background can be replaced with the C57BL/6 mouse by being back-crossed with the C57BL/6 mouse when the disease model mouse is prepared.

Preparation of an ES cell can, for example, be conducted as described below. A blastcystic embryo is collected from the uterus of a post-mated female non-human mammal [when using a mouse (preferably an inbred mouse such as C57BL/6J (B6), F₁ between B6 and another inbred line, and the like), for example, a female mouse at about 8 to about 10 weeks or so of age (about 3.5 days of gestation) mated with a male mouse at about 2 months or more of age is preferably used] (or it is also possible to collect an early embryo in the morula stage or before from the oviduct, and thereafter culture it in a medium for embryo culture in the same manner as above until the blastcyst stage), and cultured on a layer of appropriate feeder cells (for example, in the case of the mouse, a primary fibroblast prepared from a mouse fetus, a known STO fibroblast line and the like), whereby some cells of the blastcyst aggregate to form an ICM which will differentiate into an embryo. This inner cell mass is trypsinized to dissociate the single cells, and dissociation and passage are repeated while maintaining an appropriate cell density and conducting medium exchanges, whereby an ES cell is obtained.

Although the ES cell may be of either sex, a male ES cell is usually more convenient for preparation of a germ line chimera. Also, it is desirable, also for saving labor for painstaking cultivation, that sex identification be conducted as soon as possible. As an example of the ES cell sex identification method, a method wherein the gene in the sex determination region on the Y chromosome is amplified and detected by the PCR method can be mentioned. Using this method, the number of ES cells can be reduced to about 1 colony (about 50 cells), in contrast to the conventional practice that requires a cell number of about 10⁶ cells for karyotype analysis, so that primary selection of ES cells in the initial stage of cultivation can be conducted by sex identification, which in turn makes it possible to significantly save labor in the initial stage of cultivation because early selection of male cells has been made possible.

Also, secondary selection can be conducted by, for example, confirmation of the chromosome number by the G-banding method, and the like. Although the chromosome number of the ES cell obtained is desirably 100% of the normal number, it is desirable that if the 100% level is difficult to achieve for the reasons of physical operation and the like at the time of cell line establishment, gene transfer to the ES cell be followed by re-cloning into a normal cell (for example, in the case of the mouse, a cell having a chromosome number of 2n=40).

The ES cell line thus obtained need to be carefully subcultured to maintain the properties of undifferentiated stem cells. For example, a method wherein the ES cell line is cultured on appropriate feeder cells like the STO fibroblast, in the presence of LIF (1 - 10,000 U/ml), which is known as a differentiation suppression factor, in a carbon dioxide gas incubator (preferably 5% carbon dioxide gas/95% air or 5% oxygen/5% carbon dioxide gas/90% air) at about 37°C, and the like, and for passage, for example, the ES cell line is rendered to be single cells by a treatment with a trypsin/EDTA solution (usually 0.001 to 0.5% trypsin/0.1 to 5mM EDTA, preferably about 0.1% trypsin/1mM EDTA), and sown onto freshly provided feeder cells, and the like are used. This passage is usually conducted every 1 - 3 days, during which period the cells are examined; if a morphologically abnormal cell is found, the cultured cells are desirably discarded.

ES cells can be differentiated into various types of cells such as parietal muscle, visceral muscle, and myocardium by subjecting them to monolayer culture until a high density is reached, or to suspension culture until a cell aggregate is formed, under appropriate conditions [M. J. Evans and M. H. Kaufman, Nature, Vol. 292, page 154, 1981; G. R. Martin, Proc. Natl. Acad. Sci. U.S.A., Vol. 78, page 7634, 1981; T. C. Doetschman et al., Journal of Embryology and Experimental Morphology, Vol.87, page 27, 1985]; the heterologous PPARα-expressing non-human mammal cell of the present invention, obtained by differentiating an ES cell having a DNA that encodes a heterologous PPARα transferred thereto, is useful in cell biological investigations of a heterologous PPARα in vitro.

For gene transfer to an ES cell, any of the calcium phosphate co-precipitation method, electroporation method, lipofection method, retrovirus infection method, aggregation method, microinjection method, gene gun (particle gun) method, DEAE-dextran method and the like can be used; however, the electroporation method is generally chosen for the reasons of the capability of treating a large number of cells conveniently and the like. For electroporation, ordinary conditions used for gene introduction to animal cells can be used as is; for example, electroporation can be conducted by trypsinizing ES cells in the logarithmic growth phase to disperse them to obtain a dispersion of single cells, suspending the dispersion in a medium to obtain a cell density of 10⁶ to 10⁸ cells/ml, transferring the suspension to a cuvette, adding 10 to 100 µg of a vector containing a DNA that encodes the heterologous PPARα, and applying electric pulses of 200 to 600 V/cm.

Although the ES cell incorporating the transferred DNA can also be tested by screening chromosome DNAs separated and extracted from a colony obtained by culturing a single cell on feeder cells, by Southern hybridization or PCR method, the greatest advantage of a transgenic system using an ES cell resides in that a transformant can be selected at the cell stage with the expression of a drug resistance gene or a reporter gene as the index. Therefore, the introduced vector used here desirably further contains, in addition to an expression cassette containing a DNA that encodes a heterologous PPARα, a selection marker gene such as a drug resistance gene (e.g., neomycin phosphotransferase II (nptII) gene, hygromycin phosphotransferase (hpt) gene and the like) or a reporter gene (e.g., β-galactosidase (lacZ) gene, chloramphenicol acetyltransferase (cat) gene and the like). For example, when using a vector containing the nptII gene as the selection marker gene, the ES cell after gene transfer treatment is cultured in a medium containing a neomycin-series antibiotic, such as G418, each of the emerging resistant colonies is transferred to a culture plate; after trypsinization and medium exchanges are repeated, a portion thereof is reserved for cultivation, whereas the remainder is subjected to PCR or Southern hybridization to confirm the presence of the transferred DNA.

When an ES cell confirmed to have the transferred DNA incorporated therein is returned into an embryo derived from the same species of non-human mammal, it is incorporated in the ICM of the host embryo and a chimeric embryo is formed. By transplanting this to a foster mother (a female for embryo reception), and allowing development to continue, a chimeric transgenic animal is obtained. If the ES cell has contributed to the formation of primordial germ cells, which will differentiate into eggs and sperm in the chimeric animal, a germ line chimera would be obtained; by mating this, a transgenic non-human mammal having the transferred DNA fixed genetically therein can be prepared.

As the method of preparing a chimeric embryo, there are a method wherein early embryos up to the morula stage are adhered together and aggregated (aggregation chimera method) and a method wherein a cell is micro-injected into a cleavage cavity of the blastcyst (injection chimera method). Although the latter has traditionally been widely conducted in the preparation of a chimeric embryo using an ES cell, a method wherein an aggregate chimera is created by injecting an ES cell into the zona pellucida of an 8-cell stage embryo, and a method wherein an aggregate chimera is created by co-culturing and aggregating an ES cell mass and an 8-cell stage embryo with the zona pellucida removed therefrom as a method which does not require a micromanipulator and which can be easily operated, have recently also been conducted.

In all cases, a host embryo can be collected in the same manner from a non-human mammal that can be used as the female for egg collection in gene transfer to a fertilized egg; for example, in the case of the mouse, to enable a determination of the percent contribution of the ES cell to chimeric mouse formation by fur color (coat color), it is preferable to collect a host embryo from a mouse of a strain whose fur color is different from that of the strain from which the ES cell is derived. For example, provided that the ES cell is derived from the 129 strain mouse (fur color: agouti), the C57BL/6 mouse (fur color: black) or the ICR mouse (fur color: albino) can be used as the female for egg collection; provided that the ES cell is derived from the C57BL/6 or DBF₁ mouse (fur color: black) or from the TT2 cell (F₁ between C57BL/6 and CBA (fur color: agouti)), the ICR mouse or the BALB/c mouse (fur color: albino) can be used as the female for egg collection.

Also, because the potential of germ line chimera formation depends largely on the combination of ES cell and host embryo, it is more preferable to select a combination showing a high germ line chimera formation potential. For example, in the case of the mouse, it is preferable to use a host embryo and the like derived from the C57BL/6 strain for ES cells derived from the 129 strain, and host embryo and the like derived from the BALB/c strain are preferred for ES cells derived from the C57BL/6 strain.

The female mice for egg collection are preferably about 4 to about 6 weeks or so of age; as the male mouse for mating, one of the same strain at about 2 to about 8 months or so of age is preferred. Although mating may be by spontaneous mating, it is preferably conducted after gonadotropic hormones (follicle-stimulating hormone, then luteinizing hormone) are administered to induce over-ovulation.

In the case of the blast disk injection method, a blastcystic embryo (for example, in the case of a mouse, at about 3.5 days after mating) is collected from the uterus of a female for egg collection (or an early embryo in the morula stage or before, after being collected from the oviduct, may be cultured in the above-described medium for embryo culture until the blastcyst stage), and an ES cell having a DNA that encodes a heterologous PPARα transferred thereto (about 10 to about 15 cells) is injected into a cleavage cavity of the blastcyst using a micromanipulator, after which it is transplanted into the uterus of a female non-human mammal for embryo reception rendered to be pseudopregnant. As the female non-human mammal for embryo reception, a non-human mammal usable as a female for embryo reception in gene transfer to a fertilized egg can be used in the same manner.

In the case of the co-culture method, an 8-cell stage embryo and morula (for example, in the case of the mouse, about 2.5 days after mating) are collected from the oviduct and uterus of the female for egg collection (or it is also possible to collect an early embryo in the 8-cell stage or before from the oviduct, and thereafter culture it in the above-described medium for embryo culture until the 8-cell stage or the morula stage); after the zona pellucida is dissolved in acidic Tyrode's solution, an ES cell mass having a DNA that encodes a heterologous PPARα transferred thereto (number of cells: about 10 to about 15 cells) is placed in a droplet of a medium for embryo culture layered with mineral oil, the above-described 8-cell stage embryo or morula (preferably 2 cells) is further placed, and they are co-cultured overnight. The obtained morula or blastcyst is transplanted into the uterus of a female non-human mammal for embryo reception in the same manner as above.

If the transplanted embryo successfully implants and the embryo recipient female becomes pregnant, chimeric non-human mammal pups are obtained by spontaneous delivery or caesarian section. Embryo recipient females that delivered spontaneously are allowed to continue suckling; if the female has delivered by caesarian section, the pups may be suckled by a separately provided female for suckling (a female non-human mammal with usual mating and delivery).

For selection of a germ line chimera, first, a chimeric mouse, of the same sex as the ES cell, provided that the sex of the ES cell is determined in advance, is selected (usually, a male chimeric mouse is selected because a male ES cell is used), next, a chimeric mouse showing a high percent contribution of the ES cell, based on a phenotype such as fur color, is selected (for example, 50% or higher). For example, in the case of a chimeric mouse obtained from a chimeric embryo of the D3 cell, which is a male ES cell derived from the 129 strain mouse, and a host embryo derived from the C57BL/6 mouse, it is preferable to select a male mouse showing a high percentage of the agouti fur color. Whether or not the selected chimeric non-human mammal is a germ line chimera can be determined on the basis of the phenotype of the F₁ animal obtained by crossing with the same species of animal of the appropriate strain. For example, in the case of the above-described chimeric mouse, agouti is dominant over black; therefore, when the selected male mouse is crossed with a female C57BL/6 mouse, the fur color of the obtained F₁ would be agouti, provided that the selected male is a germ line chimera.

The germ line chimeric non-human mammal having a DNA that encodes a heterologous PPARα transferred thereto (founder) thus obtained is usually obtained as a heterozygote having the transferred DNA only in only one of the homologous chromosomes. Also, the individual founders are randomly inserted onto different chromosomes unless based on homologous recombination. To obtain a homozygote having a DNA that encodes a heterologous PPARα on both of the homologous chromosomes, out of F₁ animals obtained as described above, heterozygous siblings having the transferred DNA only in one of the homologous chromosomes are crossed to each other. Selection of heterozygotes can, for example, be tested by screening chromosome DNA separated and extracted from the tail of the F₁ animal by Southern hybridization or PCR method. Provided that the transferred DNA has been incorporated in only one gene locus, one-fourth of the obtained F₂ animals would be homozygotes.

The transgenic animal of the present invention is not subject to limitation as to expression of endogenous PPARα, as long as an expression amount of heterologous PPARα is assured to the extent that enables a quantitative measurement of the action of the test substance on a heterologous PPARα. However, when using a transgenic animal of the present invention to evaluate a drug that is capable of acting not only on a heterologous PPARα but also on an endogenous PPARα, it is desirable that the expression of the endogenous PPARα be inactivated. A transgenic animal of the present invention wherein the expression of endogenous PPARα has been inactivated can be obtained by introducing a DNA that encodes the heterologous PPARα, according to the method described above, to an ES cell having the PPARα gene knocked out, selected by a known method (see, for example, Lee S.S. et al., Molecular and Cellular Biology (Mol. Cell. Biol.), Vol.15, page 3012, 1995), or an early embryo or ES cell derived from a PPARα knock-out animal prepared from the ES cell according to the method described above. As a specific means of knocking out a PPARα gene, a method wherein the PPARα gene derived from the subject non-human mammal is isolated according to a conventional method, and a DNA chain having a DNA sequence constructed so that the gene is eventually inactivated by, for example, inserting another DNA fragment (for example, drug resistance genes such as the neomycin resistance gene and the hygromycin resistance gene, reporter genes such as lacZ (β-galactosidase gene) and cat (chloramphenicol acetyltransferase gene) and the like) to the exon portion thereof to destroy the function of the exon (in this case, incorporation of the transferred DNA can be selected with drug resistance or reporter gene expression as the index as described above), or by cleaving out all or a portion of the PPARα gene out using the Cre-loxP system or the Flp-frt system to delete the gene, or by inserting the stop codon into the protein-coding region to disable the complete translation of the protein, or by inserting a DNA sequence that terminates the transcription of the gene into the transcription region (for example, polyA adduct signal and the like) to disable the complete synthesis of the messenger RNA, (hereinafter abbreviated as targeting vector), is incorporated by homologous recombination into the PPARα gene locus of the subject non-human mammal, can be preferably mentioned.

Usually, gene recombinations in a mammal are mostly non-homologous; the transferred DNA is randomly inserted at an optionally chosen position on the chromosome. Therefore, it is not possible to efficiently select only those clones targeted to the target endogenous PPARα gene by homologous recombination by selection based on detection of drug resistance or reporter gene expression and the like; it is necessary to confirm the incorporation site by the Southern hybridization method or the PCR method for all selected clones. Hence, provided that, for example, the HSV-tk gene, which confers gancyclovir sensitivity, has been joined outside of a region homologous to the target sequence of the targeting vector, the cells having the vector inserted randomly thereto are incapable of growing in a gancyclovir-containing medium because they have the HSV-tk gene, whereas the cells that have been targeted to the endogenous PPARα gene locus by homologous recombination become resistant to gancyclovir and are selected because they do not have the HSV-tk gene. Alternatively, provided that the diphtheria toxin gene, for example, is joined in place of the HSV-tk gene, the cells having the vector inserted randomly thereto die due to the toxin produced thereby, so that a homologous recombinant can also be selected in the absence of a drug.

Also, the transgenic animal of the present invention having the expression of an endogenous PPARα inactivated can also be obtained by mating the thus-prepared animal having the endogenous PPARα knocked out therein and an animal of the same species having the above-described heterologous PPARα transferred thereto. For example, by further mating female and male pups obtained by mating an endogenous PPARα homo-deficient mouse and a human PPARα homo-transgenic (Tg) mouse (endogenous PPARα hetero-deficient - human PPARα hetero-Tg mouse), and selecting a mouse confirmed to have 2 copies of the human PPARα and only the deficient portion of the knocked-out mouse PPARα, it is possible to prepare an endogenous PPARα homo-deficient - human PPARα homo-Tg mouse.

Alternatively, the transgenic animal of the present invention having the expression of an endogenous PPARα inactivated may be a knock-in animal wherein an endogenous PPARα gene is substituted by a DNA that encodes a heterologous PPARα by gene targeting using homologous recombination.

A knock-in animal can be prepared according to basically the same technique as that for a knock-out animal. Because the ORF of PPARα is present in exon 3 to exon 8, for example, it is acceptable to cleave these regions of a PPARα gene derived from the subject non-human mammal using an appropriate restriction endonuclease, insert the corresponding regions of a heterologous PPARα gene instead to obtain a DNA-containing targeting vector, introduce the vector to an ES cell derived from the subject non-human mammal according to the above-described method, and select an ES cell clone having a DNA that encodes the heterologous PPARα incorporated by homologous recombination to the endogenous PPARα gene locus of the animal. Although clone selection can be conducted using the PCR method or the Southern method, for example, a homologous recombinant can be selected with drug resistance as an index, provided that a marker gene for positive selection such as the neomycin resistance gene is inserted to the 3' non-translational region and the like of the PPARα gene of the targeting vector, and a marker gene for negative selection such as the HSV-tk gene or the diphtheria toxin gene is further inserted to the outside of a region homologous to the target sequence.

Also, because there are some cases in which the expression of the heterologous PPARα having a marker gene for positive selection transferred thereto is hampered, it is preferable that a targeting vector having the loxP sequence or the frt sequence arranged at both ends of a marker gene for positive selection be used, and the marker gene for positive selection be cleaved out by allowing the Cre or Flp recombinase or an expression vector for the recombinase (e.g., adenovirus vector and the like) to act at an appropriate time after homologous recombinant selection. Alternatively, it is also acceptable to arrange a sequence homologous to the target sequence at both ends of a marker gene for positive selection repeatedly in the same direction, instead of using a Cre-loxP system or an Flp-frt system, and cleave out the marker gene for positive selection by means of intragenic recombination between the sequences.

The transgenic animal of the present invention is further characterized in that, in addition to that a DNA that encodes a heterologous PPARα is stably retained in an expressible state, it has one or more different genetic modifications. The "different genetic modification" means a genetic modification other than introduction of a DNA that encodes a heterologous PPARα, and includes a spontaneously pathogenic disease model animal having an endogenous gene modified by a spontaneous mutation, a transgenic animal having another gene further transferred thereto, a knock-out animal having an endogenous gene inactivated (including not only gene destructions due to an insertion mutation and the like, but also transgenic animals wherein the expression of a gene has been decreased to the extent that is undetectable or negligible by introduction of an antisense DNA or a DNA that encodes a neutralizing antibody), a dominant negative variant having a mutated endogenous gene transferred thereto, and the like. Therefore, a modification of an endogenous PPARα gene is also included in the scope of the "different genetic modification" in the present invention.

Although the "different genetic modification" is not subject to limitation, as long as it is advantageous for the purpose of the transgenic animal of the present invention for evaluation of the pharmacological effect of an agonist or antagonist specific for the heterologous PPARα; for example, the "different genetic modification" is preferably a genetic modification that produces a pathological condition equal or similar to the disease associated with the regulation of PPARα activity.

"A disease wherein PPARα activity regulation is involved" should be understood as a concept including not only a disease which is caused by an abnormality of PPARα activity or which eventually produces an abnormality of PPARα activity, but also a disease for which a prophylactic and/or therapeutic effect can be obtained by regulating PPARα activity. For example, as diseases that can be prevented/treated by activating a PPARα, hyperlipidemia, hypertriglyceride-(TG)-mia, combined dyslipidemia, hypo-HDL-cholesterolemia, arteriosclerosis, peripheral arterial obstruction, intermittent claudication, gangrene, hypertension, thrombosis, ischemic heart disease, acute cardiac infarction, cardiac failure, congestive cardiac failure, unstable angina pectoris, restenosis after PTCA (percutaneous transluminal coronary angioplasty), post-stenting restenosis, hyperfibrinogemia, cardiac myopathy, cerebral hemorrhage, transient cerebral ischemic stroke, cerebral infarction, stroke, chronic glomerulonephritis, diabetic nephropathy, renal arteriosclerosis, dermatitis, immunodeficiency, hypoglycemia, hypoketonemia, fatty liver, diabetes, diabetic neuropathy, diabetic retinopathy, obesity, Alzheimer's disease, anemic hypoxia, gonadal dysfunction and the like can be mentioned; as diseases that can be prevented/treated by inhibiting a PPARα, liver cancer, breast cancer and endometritis and the like can be mentioned.

As examples of the "disease model with one or more different genetic modifications that produce a pathological condition equal or similar to the disease associated with the regulation of PPARα activity", WHHL rabbit (having a mutation in the low-density lipoprotein receptor (LDLR); Watanabe Y., Atherosclerosis, Vol. 36, page 261, 1980), SHLM (a spontaneously pathogenic mouse with an apoE deletion mutation; Matsushima Y. et al., Mammalian Genome (Mamm. Genome), Vol. 10, page 352, 1999), LDLR knock-out mouse (Ishibashi S. et al., Journal of Clinical Investigation (J. Clin. Invest.), Vol. 92, page 883, 1993), apoE knock-out mouse (Piedrahita J.A. et al., Proceedings of the National Academy of Sciences (Proc. Natl. Acad. Sci. USA), Vol. 89, page 4471, 1992), human apoA, human apoB double transgenic mouse (Callow M.J. et al., Proceedings of the National Academy of Sciences, USA (Proc. Natl. Acad. Sci. USA), Vol. 91, page 2130, 1994) and the like as hyperlipidemia or arteriosclerosis models; CD55 CD59 double transgenic mouse (Cowan P.J. et al., Xenotransplantation, Vol.5, pages 184-90, 1998) and the like as ischemic heart disease models; interleukin 1 transgenic mouse (Groves R.W. et al., Proc. Natl. Acad. Sci. USA, Vol. 92, page 11874, 1995) and the like as dermatitis models; CD19 knock-out mouse (Spielman J. et al., Immunity, Vol. 3, page 39, 1995) and the like as immunodeficiency models; SPC2 knock-out mouse (Furuta M. et al., Proc. Natl. Acad. Sci. USA, Vol.94, page 6646, 1997) and the like as hypoglycemia models; ob/ob mouse (Herberg L. and Coleman D.L., Metabolism (Metabolism), Vol. 26, page 59, 1977), KK mouse (Nakamura M. and Yamada K., Diabetologia, Vol. 3, page 212, 1967), FLS mouse (Soga M. et al., Laboratory of Animal Science (Lab. Anim. Sci.), Vol. 49, page 269, 1999) as fatty liver model; NOD mouse (Makino S. et al., Experimental Animal (Exp. Anim.), Vol. 29, page 1, 1980), BB rat (Crisa L. et al., Diabetes Metabolism Review (Diabetes Metab. Rev.), Vol. 8, page 4, 1992), ob/ob mouse, db/db mouse (Hummel L. et al., Science, Vol. 153, page 1127, 1966), KK mouse, GK rat (Goto Y. et al., Tohoku Journal of Experimental Medicine (Tohoku J. Exp. Med.), Vol. 119, page 85, 1976), Zucker fatty rat (Zucker L.M. et al., Annual of the New York Academy of Science (Ann. NY Acad. Sci.), Vol. 131, page 447, 1965), OLETF rat (Kawano K. et al., Diabetes, Vol. 41, page 1422, 1992) and the like as diabetes models; ob/ob mouse, db/db mouse, KK mouse, Zucker fatty rat, OLETF rat and the like as obesity models; mutant amyloid precursor protein transgenic mouse and the like as Alzheimer's disease models; beta SAD (beta S-Antilles-D Punjab)transgenic mouse (Trudel M. et al., EMBO J, Vol. 10, page 3157, 1991) and the like as anemic hypoxia models; Steroidogenic factor 1 knock-out mouse (Zhao L. et al., Development, page 128, page 147, 2001) and the like as gonadal dysfunction models; p53 knock-out mouse (Kemp C.J., Molecular Carcinogenesis, Vol. 12, page 132, 1995) and the like as liver cancer models; c-neu transgenic mouse (Rao G.N. et al., Breast Cancer Res Treat, Vol. 48, page 265, 1998) and the like as breast cancer models; and perforin Fas-ligand double knock-out mouse (Spielman J. et al., J Immunol., Vol. 161, page 7063, 1998) and the like as endometritis models, are known.

These "disease models with different genetic modification" can, for example, be purchased from The Jackson Laboratory, USA, and the like, or can easily be prepared using a commonly known genetic modification technology.

A transgenic animal of the present invention may have been subjected to a non-genetic treatment that enables preparation of the same or another disease model, in addition to "one or more genetic modifications that produce a pathological condition equal or similar to the disease associated with the regulation of PPARα activity. "A non-genetic treatment" means a treatment that does not produce a gene modification in the subject non-human mammal. As examples of such treatments, high-fat diet loading treatment, sugar loading treatment, starvation treatment, vascular ligation/reperfusion and the like can be mentioned.

PPARα is expressed at high levels in the mammalian liver, heart, kidney, adrenal gland, gastrointestinal tract, brain and the like, and is associated with diseases in these organs. Therefore, a transgenic animal of the present invention is preferably one that specifically expresses a heterologous PPARα at 1 or 2 or more sites out of the liver, heart, kidney, adrenal gland, gastrointestinal tract and brain. Such site-specific expression can be achieved using a promoter capable of allowing the heterologous PPARα to be expressed specifically or at a high level in the target tissue described above. For example, by using the serum amyloid P component (SAP) promoter, which enables high-level expression in the liver, it is possible to prepare a transgenic animal of the present invention that expresses heterologous PPARα with liver specificity or at high levels in the liver.

Also, when expressing PPARα at a considerably high level, some non-human mammals having a DNA that encodes a heterologous PPARα transferred thereto exhibit a phenotype that develops a disease such as liver cancer, breast cancer or endometritis, without having any different genetic modification. Therefore, the present invention also provides a non-human mammal which has a DNA that encodes a heterologous PPARα transferred thereto, and which develops one or more diseases selected from the group consisting of liver cancer, breast cancer and endometritis.

As another preferred mode of the "different genetic modification" that is advantageous for the use of the transgenic animal of the present invention for evaluation of the pharmacological effect of an agonist or antagonist specific for the heterologous PPARα, introduction of a foreign DNA under the control of a PPRE-containing promoter can be mentioned. As described above, PPARα binds to the PPRE sequence present upstream of the 5' of the target gene in the presence of a ligand to promote the transcription of the gene (or to suppress the transcription in the case of some genes (e.g., apoC-III and the like)). Therefore, in a non-human mammal having a foreign DNA under the control of a PPRE-containing promoter transferred thereto, the agonist or antagonist activity of a test substance can be evaluated by determining the effect of the substance on the expression of the DNA. Here, "a foreign DNA" means a DNA gene-transferred artificially from outside, and encompasses not only heterologous DNA but also homogenic DNA. As the "foreign DNA under the control of a PPRE-containing promoter", genes derived from a human or another mammal harboring the PPRE sequence in the promoter region thereof (genomic DNA), which serve as essential target genes of PPARα, for example, genes such as CYP4A11, CYP7A1, PAI-1, ApoA-I, ApoA-II, ApoC-III and Acyl-CoA, can be mentioned. Alternatively, a chimeric DNA wherein an appropriate reporter gene (e.g., β-galactosidase gene, luciferase gene, chloramphenicol acetyltransferase gene, alkaline phosphatase gene, peroxidase gene and the like) is joined downstream of an optionally chosen promoter containing the PPRE sequence derived from these genes (functional in the cells of the subject non-human mammal) is also preferred.

The method of transferring one or more different genetic modifications that produce a pathological condition equal or similar to the disease associated with the regulation of PPARα activity, or a foreign DNA under the control of a PPRE-containing promoter, to a non-human mammal having a DNA that encodes a heterologous PPARα transferred thereto, is not subject to limitation; for example, a method wherein a non-human mammal having a DNA that encodes a heterologous PPARα transferred thereto and the same species of non-human mammal having one or more different genetic modifications that produce a pathological condition equal or similar to the disease associated with the regulation of PPARα activity, or a foreign DNA under the control of a PPRE-containing promoter, are crossed; a method wherein a DNA that encodes a heterologous PPARα is transferred, by the above-described method, to an early embryo or ES cell of a non-human mammal having one or more different genetic modifications that produce a pathological condition equal or similar to the disease associated with the regulation of PPARα activity, or a foreign DNA under the control of a PPRE-containing promoter, to obtain a transgenic animal; a method wherein one or more different genetic modifications that produce a pathological condition equal or similar to the disease associated with the regulation of PPARα activity, or a foreign DNA under the control of a PPRE-containing promoter, is transferred to an early embryo or ES cell of a non-human mammal having a DNA that encodes a heterologous PPARα transferred thereto, by the above-described method, or by a knock-out technology; and the like can be mentioned. Also, when one or more different genetic modifications are by introduction of a foreign gene or dominant mutation gene that produces a pathological condition equal or similar to the disease associated with the regulation of PPARα activity, it is possible to obtain a transgenic animal by simultaneously or sequentially transferring the foreign gene and the like and a DNA that encodes a heterologous PPARα transgenic animal, to an early embryo or ES cell of a wild type on-human mammal. When different genetic modification is introduction of a foreign DNA under the control of a PPRE-containing promoter as well, it is possible to obtain a transgenic animal by simultaneously or sequentially introducing the foreign DNA and a DNA that encodes a heterologous PPARα. Furthermore, when one or more different genetic modifications that produce a pathological condition equal or similar to the disease associated with the regulation of PPARα activity are by destruction of an endogenous gene, it is acceptable to design a DNA that encodes a heterologous PPARα to make it capable of targeting the endogenous gene to be destroyed, and to transfer it to an ES cell of a wild type non-human mammal. In this case, as the targeting vector, the same as those mentioned as examples with respect to the preparation of the above-described knock-in animal, except that the endogenous PPARα gene is replaced with the endogenous gene to be destroyed, can be preferably used.

When crossing a non-human mammal having a DNA that encodes a heterologous PPARα and the same species of a disease model non-human mammal having one or more different genetic modifications that produce a pathological condition equal or similar to the disease associated with the regulation of PPARα activity (or a foreign DNA under the control of a PPRE-containing promoter) are crossed, it is desirable to cross homozygotes with each other. For example, the F₁ obtained by crossing a homozygote having a DNA that encodes a heterologous PPARα incorporated in one gene locus thereof and an apoE homo-deficient hyperlipidemia (arteriosclerosis) model is hetero with respect to both genes. One-sixteenth of the F₂ animals obtained by sibling mating of such F₁ animals are heterologous PPARα homo-transgenic - apoE homo-deficient.

Because "the transgenic non-human mammal of the present invention" obtained as described above expresses, in addition to an endogenous PPARα (or in place thereof), a heterologous PPARα, it enables an evaluation in vivo of the pharmacological effect of an agonist or antagonist specific for a heterologous PPARα which possesses agonist or antagonist activity against a heterologous PPARα, but which does not possess activity against an endogenous PPARα. Therefore, the present invention provides a screening method for a heterologous PPARα agonist or antagonist, characterized in that a test substance is applied to the transgenic non-human mammal of the present invention or a portion of the body thereof, and the agonist or antagonist activity of the substance against a heterologous PPARα is tested.

Here, "agonist activity" refers to a property for specifically binding to a PPARα to shift the equilibrium state between the active form (that is, a state wherein the agonist, as a heterodimer with RXR, is capable of binding to PPRE to activate the transcription of the target gene) and the inactive form of the PPARα toward higher activity, with the degree thereof being not subject to limitation. Therefore, the scope of "a substance possessing agonist activity (agonist)" also encompasses partial agonists, as well as what is called full agonists. On the other hand, "antagonist activity" refers to a property for antagonistically binding to the ligand-binding site of a PPARα, but having no or almost no influence on the equilibrium state between the active form and the inactive form, or a property for binding to an optionally chosen site of a PPARα to shift the equilibrium state between the active form and the inactive form of the PPARα toward lower activity. Therefore, "a substance possessing antagonist activity (antagonist)" as used in the present specification is understood to be defined as a concept encompassing both what is called neutral antagonist and inverse agonist.

Specifically, in the screening method of the present invention, a test substance is administered to the transgenic non-human mammal of the present invention. As the test substance, in addition to a known synthetic compound, peptide, protein, DNA library and the like, for example, tissue extract, cell culture supernatant and the like of a mammal (for example, mouse, rat, swine, bovine, sheep, monkey, human and the like) are used. It is desirable to confirm in advance that the test substance acts specifically on the heterologous PPARα, by conducting a binding experiment in vitro with each of an endogenous PPARα and heterologous PPARα of the transgenic non-human mammal of the present invention to confirm binding only to the heterologous PPARα, or by conducting an expression assay in each of a non-human mammal-derived cell and a heterologous mammal-derived cell, each having a reporter gene under the control of PPRE introduced thereto, to confirm activation of the expression of the reporter gene only in the latter, and the like. Provided that the endogenous PPARα has been knocked out in the transgenic non-human mammal of the present invention, this preliminary screening can be omitted.

The PPARα agonist/antagonist activity of the test substance can, for example, be tested with fatty acid β oxidation activity or accompanying changes in blood TG concentration, apoC-III production or accompanying changes in blood TG concentration, apoA-I production or accompanying changes in blood HDL-C concentration, apoA-II production or accompanying changes in blood HDL-C concentration, and the like as indices. These can be measured in accordance with a method known per se, for example, the method described in Chung, B.H., Segrest, J.P., Ray, M.J., Brunzell, J.D., Hokanson, J.E., Krauss, R.M., Beaudrie, K., and Cone, J.T., 1986. Methods Enzymol. 128: 181-209. In the transgenic non-human mammal of the present invention, wherein "different genetic modification" is "introduction of a foreign DNA under the control of a PPRE-containing promoter", the PPARα agonist/antagonist activity of the test substance can easily be measured by examining the expression of the foreign DNA.

The thus-selected heterologous PPARα agonist can be used as a safe low-toxicity prophylactic/therapeutic drug in the animal from which the heterologous PPARα is derived, for diseases such as hyperlipidemia, hypertriglyceridemia, combined dyslipidemia, hypo-HDL-cholesterolemia, arteriosclerosis, peripheral arterial obstruction, intermittent claudication, gangrene, hypertension, thrombosis, ischemic heart disease, acute myocardial infarction, heart failure, congestive heart failure, unstable angina pectoris, post-PTCA restenosis, post-stenting restenosis, hyperfibrinogemia, cardiomyopathy, cerebral hemorrhage, transient cerebral ischemic attack, cerebral infarction, cerebral apoplexy, chronic glomerulonephritis, diabetic nephropathy, renal arteriosclerosis, dermatitis, immunodeficiency, hypoglycemia, hypoketonemia, fatty liver, diabetes mellitus, diabetic neuropathy, diabetic retinopathy, obesity, Alzheimer's disease, anemic hypoxia and gonadal dysfunction.

The heterologous PPARα agonist can, for example, be used orally as a tablet, capsule, elixir, microcapsule and the like, with a sugar coating made as necessary, or parenterally in the form of a sterile solution with water or another pharmaceutically acceptable solution, or an injection such as a suspension. The agonist can be formulated into a preparation by being admixed in a unit dose form required for generally recognized preparation making, along with a physiologically acceptable carrier, a flavoring agent, a filler, a vehicle, an antiseptic, a stabilizer, a binder and the like. The active ingredient content in these preparations is appropriately chosen in consideration of the dosage described below.

As examples of additives that can be admixed in tablets, capsules and the like, binders like gelatin, corn starch, tragacanth, and acacia; fillers like crystalline cellulose; swelling agents like corn starch, gelatin, and alginic acid; lubricants like magnesium stearate; sweetening agents like sucrose, lactose or saccharin; flavoring agents like peppermint, Gaultheria adenothrix oil or cherry; and the like are used. When the formulation unit form is a capsule, the above-described type of material can further contain a liquid carrier like an oil or fat. A sterile composition for injection can be formulated according to an ordinary preparation design such as dissolving or suspending an active substance, a naturally produced vegetable oil such as sesame oil or coconut oil, and the like in a vehicle like water for injection. As examples of aqueous solutions for injection, physiological saline, an isotonic solution containing glucose or other auxiliary drugs (for example, D-sorbitol, D-mannitol, sodium chloride and the like) and the like can be mentioned, which may be used in combination with an appropriate solubilizer, for example, an alcohol (for example., ethanol and the like), a polyalcohol (for example, propylene glycol, polyethylene glycol and the like), a non-ionic surfactant (for example, polysorbate 80™, HCO-50 and the like),and the like. As examples of oily solutions, sesame oil, soybean oil and the like can be mentioned, which may be used in combination with solubilizers benzyl benzoate, benzyl alcohol and the like. Also, the heterologous PPARα agonist may be formulated with a buffering agent (for example, phosphate buffer solution, sodium acetate buffer solution and the like), a soothing agent (for example, benzalkonium chloride, procaine hydrochloride and the like), a stabilizer (for example, human serum albumin, polyethylene glycol and the like), a preservative (for example, benzyl alcohol, phenol and the like), an antioxidant and the like. The prepared injection solution is usually filled in an appropriate ampoule.

Also, when the heterologous PPARα agonist is a nucleic acid such as a DNA or an RNA, the nucleic acid, alone, or after the DNA (or DNA corresponding to the RNA) is inserted to an appropriate vector such as retrovirus vector, adenovirus vector, or adenovirus-associated virus vector, can be administered to a human or another mammal according to a standard means. The nucleic acid, as is, or after being formulated into a preparation along with a physiologically acceptable carrier such as an auxiliary for promotion of ingestion, can be administered using a gene gun or a catheter like a hydrogel catheter.

Because the preparation thus obtained is safe and of low toxicity, it can be administered to, for example, a mammal having the same or substantially the same PPARα as the heterologous PPARα (for example, human, rat, mouse, guinea pig, rabbit, sheep, swine, bovine, horse, cat, dog, monkey and the like; "substantially the same" has the same definition as that given above), preferably an animal from which the heterologous PPARα is derived (preferably human).

The dosage of the heterologous PPARα agonist varies depending on the target disease, the subject of administration, the route of administration and the like; for example, when the agonist is orally administered for the purpose of treating hyper-TG-emia, the dosage in an adult (body weight 60 kg) is generally about 0.1 mg to 100 mg, preferably about 1.0 to 50 mg, more preferably about 1.0 to 20 mg per day. In the case of parenteral administration, the dosage of the agonist varies depending on the subject of administration, target disease and the like; for example, when the agonist is administered for the purpose of treating hyper-TG-emia as an injection to an adult (body weight 60 kg), the dosage is about 0.01 to 30 mg or so, preferably about 0.1 to 20 mg or so, more preferably about 0.1 to 10 mg or so per day. When the subject of administration is a non-human animal as well, a dosage calculated per 60 kg body weight can be administered.

Also, a heterologous PPARα antagonist selected by the screening method of the present invention can be used as a safe low-toxicity prophylactic/therapeutic drug in a mammal (preferably a mammal from which the heterologous PPARα is derived, more preferably human), for diseases such as liver cancer, breast cancer or endometritis. The heterologous PPARα antagonist can be formulated into a preparation by the same method as that for the above-described heterologous PPARα agonist, and can be orally or parenterally administered to a mammal (for example, human, rat, mouse, guinea pig, rabbit, sheep, swine, bovine, horse, cat, dog, monkey and the like; "substantially the same" has the same definition as that given above).

The dosage of the heterologous PPARα antagonist varies depending on target disease, the subject of administration, the route of administration and the like; for example, when the antagonist is orally administered for the purpose of treating liver cancer, the dosage in an adult (body weight 60 kg) is generally about 0.1 mg to 100 mg, preferably about 1.0 to 50 mg, more preferably about 1.0 to 20 mg, per day. In the case of parenteral administration, the dosage of the antagonist varies depending on the subject of administration, target disease and the like; for example, when the antagonist is administered for the purpose of treating liver cancer as an injection to an adult (body weight 60 kg), the dosage is about 0.01 to 30 mg or so, preferably about 0.1 to 20 mg or so, more preferably about 0.1 to 10 mg or so, per day. When the subject of administration is a non-human animal as well, a dosage calculated per 60 kg body weight can be administered.

Because "the transgenic non-human mammal of the present invention" produces a pathological condition equal or similar to the disease associated with the regulation of PPARα activity, it is possible to screen for a substance possessing prophylactic/therapeutic activity against a disease in a mammal (preferably human) from which the heterologous PPARα is derived, and in which the regulation of the activity of the PPARα is involved, by administering a test substance to the animal, and testing the effect of the substance on the pathological condition. The pathological condition serving as an index can be appropriately selected depending on the kind of disease model; for example, the prophylactic/therapeutic effect of the test substance against disease can be evaluated with an improvement in blood cholesterol concentration (total cholesterol concentration, TG concentration, HDL-C concentration and the like) and the like as the index when the transgenic non-human mammal of the present invention is a hyperlipidemia model, and with arteriosclerosis lesion involution and the like as the index when the same is an arteriosclerosis model, blood flow improvement and the like as the index when the same is a model of ischemic heart disease or cerebral vascular disease, and with cancer focus involution and the like as the index when the same is a model of liver cancer or breast cancer.

As described above, when expressing PPARα at a considerably high level, some non-human mammals having a DNA that encodes a heterologous PPARα transferred thereto exhibit a phenotype that develops a disease such as liver cancer, breast cancer or endometritis, without having any different genetic modification. Therefore, by administering the test substance to such a heterologous PPARα-transferred non-human mammal in the same way, and testing the effect to ameliorate the pathological condition of the above-described disease, substances possessing prophylactic/therapeutic activity for the disease can also be screened for.

The thus selected substance can be used as a safe low-toxicity prophylactic/therapeutic drug for diseases such as hyperlipidemia, hypertriglyceridemia, combined dyslipidemia, hypo-HDL-cholesterolemia, arteriosclerosis, peripheral arterial obstruction, intermittent claudication, gangrene, hypertension, thrombosis, ischemic heart disease, acute myocardial infarction, heart failure, congestive heart failure, unstable angina pectoris, post-PTCA restenosis, post-stenting restenosis, hyperfibrinogemia, cardiomyopathy, cerebral hemorrhage, transient cerebral ischemic attack, cerebral infarction, cerebral stroke, chronic glomerulonephritis, diabetic nephropathy, renal arteriosclerosis, dermatitis, immunodeficiency, hypoglycemia, hypoketonemia, fatty liver, diabetes, diabetic neuropathy, diabetic retinopathy, obesity, Alzheimer's disease, anemic hypoxia, gonadal dysfunction, liver cancer, breast cancer and endometritis. the selected substance can be formulated into a preparation by the same method as that for the above-described heterologous PPARα agonist, and orally or parenterally administered to a mammal (for example, human, rat, mouse, guinea pig, rabbit, sheep, swine, bovine, horse, cat, dog, monkey and the like).

The dosage of the selected substance varies depending on target disease, the subject of administration, the route of administration and the like; for example, when the substance is orally administered for the purpose of treating hyper-TG-emia, the dosage in an adult (body weight 60 kg) is generally about 0.1 mg to 100 mg, preferably about 1.0 to 50 mg, more preferably about 1.0 to 20 mg per day. In the case of parenteral administration, the dosage of the substance varies depending on the subject of administration, target disease and the like; for example, when the substance is administered for the purpose of treating hyper-TG-emia as an injection to an adult (body weight 60 kg), the dosage is about 0.01 to 30 mg or so, preferably about 0.1 to 20 mg or so, more preferably about 0.1 to 10 mg or so per day. When the subject of administration is a non-human animal as well, a dosage calculated per 60 kg body weight can be administered.
Sequence identification numbers in the sequence listing in the present specification indicate the following sequences.
[SEQ ID NO :1] Shows the base sequence of cDNA encoding human PPARα.
[SEQ ID NO :2] Shows the amino acid sequence of human PPARα.
[SEQ ID NO :3] Shows the base sequence of an oligonucleotide designed to function as a primer for amplifying human SAP promoter.
[SEQ ID NO :4] Shows the base sequence of an oligonucleotide designed to function as a primer for amplifying human SAP promoter.
[SEQ ID NO :5] Shows the base sequence of an oligonucleotide designed to function as a primer for amplifying rabbit β-globin enhancer.
[SEQ ID NO :6] Shows the base sequence of an oligonucleotide designed to function as a primer for amplifying rabbit β-globin enhancer.
[SEQ ID NO :7] Shows the base sequence of an oligonucleotide designed to function as a primer for amplifying cDNA fragment of human PPARα.
[SEQ ID NO :8] Shows the base sequence of an oligonucleotide designed to function as a primer for amplifying cDNA fragment of human PPARα.
[SEQ ID NO :9] Shows the base sequence of an oligonucleotide designed to function as a fluorescent probe to detect cDNA fragment of human PPARα amplified by PCR.

When bases, amino acids and the like are shown in abbreviations in the present specification, they are based on the abbreviations by the IUPAC-IUB Commission on Biochemical Nomenclature or abbreviations conventionally used in the pertinent field; examples thereof are given below.
DNA: Deoxyribonucleic acid
cDNA: Complementary deoxyribonucleic acid
A: Adenine
T: Thymine
G: Guanine
C: Cytosine
RNA: Ribonucleic acid
mRNA: Messenger ribonucleic acid
dATP: Deoxyadenosine triphosphate
dTTP: Deoxythymidine triphosphate
dGTP: Deoxyguanosine triphosphate
dCTP: Deoxycytidine triphosphate
ATP: Adenosine triphosphate
EDTA: Ethylenediamine tetraacetate
SDS: Sodium dodecyl sulfate
Gly: Glycine
Ala: Alanine
Val: Valine
Leu: Leucine
lie Isoleucine
Ser: Serine
Thr: Threonine
Cys: Cysteine
Met: Methionine
Glu: Glutamic acid
Asp: Aspartic acid
Lys: Lysine
Arg: Arginine
His: Histidine
Phe: Phenylalanine
Tyr: Tyrosine
Trp: Tryptophan
Pro: Proline
Asn: Asparagine
GIn: Glutamine
pGlu: Pyroglutamic acid
Me: Methyl group
Et: Ethyl group
Bu: Butyl group
Ph: Phenyl group
TC: Thiazolyzine-4 (R)-carboxamide group

The present invention is described in more detail by means of the following Examples, which are not to be construed as limiting the scope of the present invention.

### Example 1: Construction of PPARα vector

The hSAP (human serum amyloid P component) promoter, which is necessary for high-level expression of the introduced gene in the liver was cloned by PCR. From the human genome data in GENBANK, it is known that hSAP is present on chromosome 1. Since the 5' upstream region could be identified by search of homology with the cDNA sequence, the primers SA1 (5'-ACTGAGTAGAAGTAGCAGAA-3' (SEQ ID NO: 3)) and SA4 (5'-CAGCGGCTTGTTCATATTCC-3' (SEQ ID NO: 4)) were designed on the basis of the genome sequence of a promoter region used in the literature (J. Biol. Chem., 111, 736, 1992; Dev. Genet., 10, 336, 1989), a 0.67 Kbp HindIII-AvrII fragment adjoining to the initiation codon was amplified by PCR (Takara Shuzo Ex Taq DNA polymerase used; treatment at 94°C for 2 minutes followed by treatment at 94°C for 0.5 minutes, at 60°C for 0.5 minutes, and at 68°C for 1 minute in 30 cycles), the obtained fragment was cloned into the pCR2.1 vector (invitrogen Company) by the TA cloning (invitrogen Company) method, and the sequence was identified. Four clones were analyzed, and one clone lacking base substitution was obtained (plasmid name: pCR2.1-SA4).

Next, cloning of a rabbit β-globin enhancer that is necessary for high-level expression in host mammalian cells was conducted by the method described below. With genomic DNA prepared from rabbit blood as the template, and using primers having a restriction endonuclease site for expression vector construction added to the 5' terminus thereof, namely BG2 (5'-TCCTAGGTGAGAACTTCAGGGTGAGTTTG-3' (SEQ ID NO: 5); with an AvrII site added) and BG3 (5'-CGGTACCTTTGCCAAAATGATGAGACAGC-3' (SEQ ID NO: 6); with a KpnI site added), PCR (Takara Shuzo Ex Taq DNA polymerase used; treatment at 94°C for 2 minutes followed by treatment at 94°C for 0.5 minutes, at 60°C for 0.5 minutes, and at 72°C for 1 minute in 30 cycles) was conducted to obtain an about 640 bp amplified enhancer region. The amplified fragment was cloned into the pCR2.1-TOPO vector by TA cloning (invitrogen Company); 8 clones were examined for sequence confirmation (ABI Company BigDye Terminator used). As a result of a comparison of the sequence analysis results for the 8 clones with enhancer sequences, the sequences of the 8 clones generally agreed with known sequences, so that they were judged to be derived from the rabbit genome of the PCR template.

The PPARα cDNA used was a 1.4 Kbp KpnI-SalI fragment cleaved out from pMCMV-neo-hPPARα (Biochem. Biophys. Res. Commun., 278: 704-711 (2000)). For SV40 polyA, the fragment from BglII to HindIII containing the polyA adduct signal derived from pTB399 (R. Sasada et al., Cell Structure and Function, 12: 205, 1987) was cloned into pSP73 (stratagene Company); after an Sail linker was added to the BglII site, the fragment was used as a 0.27 Kbp SalI-HindIII fragment.

In constructing an expression vector, fist, an AvrII-KpnI fragment recovered from pCR2.1-enh5 was ligated to the AvrII-KpnI site of pCR2.1-SA4 using a Takara ligation kit (Takara Shuzo) to yield pCR2.1-SAPENH1 incorporating the promoter and the enhancer. On the other hand, an about 1.4 Kbp hPPARα cDNA prepared from pMCMV-neo-hPPARα and an about 0.27 Kbp SV40 polyA fragment (R. Sasada et al., Cell Structure and Function, 12: 205, 1987) were ligated to the KpnI-HindIII site of pBluescriptII KS- (stratagene) to yield pKS-PPAR polyAl incorporating the cDNA and polyA. Subsequently, a NotI-KpnI fragment recovered from pCR2.1-SAPENH1 (about 1.3 Kbp) and a KpnI-NotI fragment recovered from pMCMV-neo-hPPARα (about 1.7 Kbp) were ligated to the NotI site of pBluescriptII KS-(stratagene) to complete the construction of the expression vector (plasmid name: pKS-SEPP2; a transformant obtained by cloning this plasmid into the Escherichia coli JM109 strain (Escherichia coli JM109/pKS-SEPP2) has been deposited under accession number FERM BP-8151, since August 14, 2002, at the International Patent Organism Depositary, National Institute of Advanced Industrial Science and Technology at Central 6, 1-1-1, Higashi, Tsukuba, Ibaraki, Japan (zip code: 305-8566).). The injection fragment used to prepare a transgenic mouse was cleaved out by cleavage with NotI, and has a size of about 3.0 Kbp (Figure 1).

### Example 2: Preparation of transgenic mouse having human PPARα transferred thereto

To female mice for egg collection (C57BL/6J strain, 9 - 13 weeks of age), 5 IU PMSG was intraperitoneally administered; after the animals were reared in a breeding room with a 12 hours light period/12 hours dark period for 2 days, 5 IU hCG was intraperitoneally administered, and they were mated with male mice (C57BL/6J strain, 15 to 20 weeks old). Separately, spontaneously estrual females for embryo reception mice (ICR strain, 9 to 20 weeks old) were mated with vasoligated male mice (ICR strain, 15 to 20 weeks old). On the following day, the abdominal cavity of each female mouse for egg collection with copulation confirmed by vaginal plug formation was opened and the oviduct was taken out; in M2 medium, fertilized eggs were taken out using tweezers under a stereoscopic microscope. A fertilized egg from which cumulus cells had left was aspirated using a pipette, placed in a drop of M16 medium covered with mineral oil, and cultured at 37°C in the presence of 5% CO₂ for 2 hours until injection.

A fertilized egg was placed in a drop of M2 medium covered with mineral oil, and aspirated and immobilized using a holding pipette. The injection fragment solution (0.3 to 1.0 µg/ml) prepared in Example 1 was aspirated into an injection pipette, the male pronucleus of the fertilized egg was pricked with the injection pipette under a stereoscopic microscope, and the injection fragment was injected. After completion of the injection, the fertilized egg was placed in a drop of M2 medium covered with mineral oil, and cultured at 37°C in the presence of 5% CO₂ until transplantation to a female for embryo reception.

After a female for embryo reception mouse confirmed by vaginal plug formation to be in a pseudopregnant state was anesthetized with Nembutal, an incision was made in the posterior back, a fat mass was picked and drawn out using tweezers, and immobilized using forceps. The ovarian sac was torn using tweezers under a stereoscopic microscope, and 10 to 15 fertilized eggs per each oviduct were injected to the oviduct opening using a transfer pipette. The ovary and the oviduct were returned into the body and the incision was sutured, after which the animal was continued to be reared in a breeding room with 12-hour light period/12-hour dark period. At 21 days after embryo transplantation, mouse pups (F₀) were born. The transmission of the transferred DNA to the mouse pups was confirmed by cutting out an about 1 cm portion of the tail using scissors, isolating DNA from the tissue extract by a conventional method, and conducting the PCR method.

The F₀ animals confirmed as having the transferred DNA transmitted thereto were mated with the C57BL/6J mouse at a stage when they became capable of reproduction, and pups (F₁) were obtained. The transmission of the transferred DNA was confirmed in the same manner as above, and F₁ animals having the transferred DNA (siblings) were mated to obtain animals homozygous with respect to the transferred DNA.

### Example 3: Gene expression analysis of transgenic mouse

To examine the human PPARα expression level in the transgenic mouse obtained in Example 2, the liver was collected from the obtained transgenic mouse at 5 to 6 weeks old, and total RNA was extracted using ISOGEN (manufactured by Nippon Gene Co., Ltd.). Next, on the basis of a total RNA purified using an RNase-free DNase set (manufactured by QIAGEN K.K.) and the RNeasy Mini Kit (manufactured by QIAGEN K.K.), a cDNA was prepared by a reverse transcription reaction using the kit TaqMan Transcription Reagents (manufactured by Applied Biosystems Company). As the primer for real-time PCR (TaqMan PCR), 5'-CGCCAGCACGGACGA-3' (SEQ ID NO: 7) and 5'-TTGTCCCCACATATTCGACACTC-3' (SEQ ID NO: 8) were used, and as the FAM (fluorescent 5-carboxyfluorescein)-labeled TaqMan probe, 5'-CCCCCGGCAGTGCCCTGAA-3' (SEQ ID NO: 9) was used. Furthermore, using the TaqMan PCR Master Mix (manufactured by Applied Biosystems Company), a Real-time PCR REACTION was conducted in a dedicated plate with 20 µl of each cDNA sample as the template. The reaction was conducted using the PCR apparatus 7700 sequence detector (manufactured by Applied Biosystems Company) by a treatment at 50°C for 2 minutes and at 95°C for 10 minutes followed by a treatment at 95°C for 15 seconds and at 60°C for 1 minute in 40 cycles. After the reaction, data analysis was conducted according to the analytical manual. As a standard for the quantitation described above, expression of the GAPDH gene was quantified using the same cDNA sample, and using the TaqMan Rodent GAPDH Control Reagent (manufactured by Applied Biosystems Company), and the results were used for data value correction. As a result of the experiment, the expression of human PPARα was confirmed in the liver of the transgenic mouse.

### Example 4: Mating with PPARα homo-deficient mouse

The human PPARα homo-Tg mouse prepared in Example 2 and a PPARα homo-deficient mouse (The Jackson Laboratory, USA) are mated to obtain pups. DNA is extracted from the tail of each mouse pup and subjected to Southern blotting analysis; a parent mouse that delivered pups in all of which the human PPARα and the knocked-out deficient portion of the mouse PPARα gene were detected, is selected as a human PPARα hetero-Tg - mouse PPARα hetero-deficient mouse. Next, the female and the male are mated. Gene identification is conducted by the same method; a mouse confirmed to have 2 copies of the human PPARα and only the deficient portion of the knocked-out mouse PPARα gene is selected as a human PPARα homo-Tg - mouse PPARα homo-deficient mouse.

### Example 5: Mating with various hyperlipidemia (arteriosclerosis) model mice

### (1) Mating with apoE homo-deficient mouse

Female and male pups obtained by mating the human PPARα homo-Tg mouse prepared in Example 2 and an apoE homo-deficient mouse that is a model of hyperlipemic (arteriosclerotic) model (The Jackson Laboratory, USA; having C57BL/6J as the genetic background) are mated. DNA is extracted from the tail of each obtained mouse pup in the same manner as above, and subjected to Southern blotting analysis; DNA is extracted from the tail of each obtained mouse pup and subjected to Southern blotting analysis; a parent mouse that delivered pups in all of which the DNA of the human PPARα and the knocked-out apoE gene were detected, is selected as a human PPARα hetero-Tg - apoE hetero-deficient mouse. Next, the female and the male are mated. Gene identification is conducted by the same method; a mouse confirmed to have 2 copies of the human PPARα and only the deficient portion of the knocked-out apoE gene is selected as a human PPARα homo-Tg - apoE homo-deficient mouse.

### (2) Mating with LDL receptor homo-deficient mouse

The human PPARα homo-Tg mouse prepared in Example 2 and an LDL receptor homo-deficient mouse that is a hyperlipidemic (arteriosclerotic) disease model [a mouse prepared by Takeda Chemical Industries, Ltd. (described in Japanese Laid-Open Patent Publication No. 10-56915), or a mouse stored by The Jackson Laboratory, USA; both have C57BL/6J as the genetic background] are mated to obtain pups. DNA is extracted from the tail of each obtained mouse pup in the same manner as above and subjected to Southern blotting analysis; a parent mouse that delivered pups in all of which the DNA of the human PPARα and the knocked-out LDL receptor gene were detected, is selected as a human PPARα hetero-Tg - LDL receptor hetero-deficient mouse. Next, the female and the male are mated. Gene identification is conducted by the same method; a mouse confirmed to have 2 copies of the human PPARα and only the deficient portion of the knocked-out LDL receptor gene is selected as a human PPARα homo-Tg - LDL receptor homo-deficient mouse.

### (3) Mating with human apoA-I homo-Tg mouse

Females and males of pups obtained by mating the human PPARα homo-Tg mouse prepared in Example 2 and a human apoA-I homo-Tg mouse that is a model of hyperlipemic (arteriosclerotic) model (The Jackson Laboratory, USA; having C57BL/6J as the genetic background) are mated. DNA is extracted from the tail of each obtained mouse pup in the same manner as above and subjected to Southern blotting analysis; a parent mouse that delivered pups in all of which the DNA of the human PPARα and the human apoA-I gene were detected, is selected as a human PPARα - human apoA-I hetero-Tg mouse. Next, the female and the male are mated. Gene identification of the obtained pups is conducted by the same method; a mouse confirmed to have 2 copies of each of the human PPARα and human apoA-I is selected as a human PPARα - human apoA-I homo-Tg mouse.

### (4) Furthermore, in each of (1) to (3) above, the human PPARα homo-Tg - mouse PPARα homo-deficient mouse prepared in Example 4, in place of the human PPARα homo-Tg mouse prepared in Example 2, is mated with each of the apoE homo-deficient mouse, the LDL receptor homo-deficient mouse and the human apoA-I Tg mouse through the same procedures.

### Industrial Applicability

The heterologous PPARα-transferred disease model non-human mammal of the present invention provides a screening system useful in the development of prophylactic/therapeutic drugs for various diseases wherein PPARα activity regulation is involved as it enables an evaluation of the in vivo pharmacological effect of a PPARα agonist or antagonist that acts on the heterologous PPARα but does not act on endogenous PPARα of the non-human mammal.

### Free Texts in Sequence Listing

SEQ ID NO :3
   shows an oligonucleotide designed to function as a primer for amplifying human SAP promoter.
SEQ ID NO:4
   shows an oligonucleotide designed to function as a primer for amplifying human SAP promoter.
SEQ ID NO:5
   shows an oligonucleotide designed to function as a primer for amplifying rabbit β-globin enhancer.
SEQ ID NO:6
   shows an oligonucleotide designed to function as a primer for amplifying rabbit β-globin enhancer.
SEQ ID NO:7
   shows an oligonucleotide designed to function as a primer for amplifying cDNA fragment of human PPARα.
SEQ ID NO:8
   shows an oligonucleotide designed to function as a primer for amplifying cDNA fragment of human PPARα.
SEQ ID NO:9
   shows an oligonucleotide designed to function as a fluorescent probe to detect cDNA fragment of human PPARα amplified by PCR.

## Claims

1. A non-human mammal, which stably retains a DNA encoding a heterologous PPARα in an expressible state and has one or more different genetic modifications, or a part of its living body.

2. The animal or the part of its living body of claim 1, wherein at least one of said different genetic modifications results in pathological condition(s) equal or similar to disease(s) associated with the regulation of PPARα activity.

3. The animal or the part of its living body of claim 1, wherein at least one of said different genetic modifications is introduction of a foreign DNA under the control of a promoter having PPRE.

4. The animal or the part of its living body of claim 1, wherein said heterologous PPARα is human derived PPARα.

5. The animal or the part of its living body of claim 1, wherein said heterologous PPARα has the same or substantially the same amino acid sequence as the amino acid sequence represented by SEQ ID NO: 2.

6. The animal or the part of its living body of claim 1, wherein said non-human mammal is rabbit, dog, cat, guinea pig, hamster, mouse or rat.

7. The animal or the part of its living body of claim 1, wherein said non-human mammal is mouse.

8. The animal or the part of its living body of claim 1, wherein said animal expresses said heterologous PPARα in place of lacking its endogenous PPARα.

9. The animal or the part of its living body of claim 8, wherein said animal is obtainable by crossing an endogenous PPARα-deficient animal and the same species of animal that expresses a heterologous PPARα.

10. The animal or the part of its living body of claim 8, wherein said endogenous PPARα is mouse-derived PPARα and said heterologous PPARα is human-derived PPARα.

11. The animal or the part of its living body of claim 2, wherein said diseases associated with the regulation of PPARα activity are one or more diseases selected from the group consisting of hyperlipidemia, hypertriglyceridemia, combined dyslipidemia, hypo-HDL-cholesterolemia, arteriosclerosis, peripheral arterial obstruction, intermittent claudication, gangrene, hypertension, thrombosis, ischemic heart disease, acute myocardial infarction, heart failure, congestive heart failure, unstable angina pectoris, post-PTCA restenosis, post-stenting restenosis, hyperfibrinogemia, cardiomyopathy, cerebral hemorrhage, transient ischemic attack, cerebral infarction, cerebral apoplexy, chronic glomerulonephritis, diabetic nephropathy, renal arteriosclerosis, dermatitis, immunodeficiency, hypoglycemia, hypoketonemia, fatty liver, diabetes mellitus, diabetic neuropathy, diabetic retinopathy, obesity, Alzheimer's disease, anemic hypoxia, gonadal dysfunction, liver cancer, breast cancer and endometritis.

12. The animal or the part of its living body of claim 1, wherein said heterologous PPARα is specifically expressed in one or more region selected from the group consisting of liver, heart, kidney, adrenal gland, blood vessel, gastrointestinal tract and brain.

13. The animal or the part of its living body of claim 1, wherein said heterologous PPARα is specifically expressed in liver.

14. A method of screening for an agonist or antagonist for a heterologous PPARα, which comprises applying a test substance to the animal or the part of its living body of claim 1, and assaying its agonistic or antagonistic activity against the heterologous PPARα.

15. A method of screening for an agonist or antagonist for a heterologous PPARα, which comprises applying a test substance to the animal or the part of its living body of claim 3, and assaying its agonistic or antagonistic activity against the heterologous PPARα using the expression of a foreign DNA under the control of a promoter having PPRE as an index.

16. A method of screening for a substance having a prophylactic/therapeutic activity for disease(s) associated with the regulation of PPARα activity in an animal from which a heterologous PPARα is derived, which comprises administering a test substance to the animal of claim 2, and assaying effect(s) of the substance on pathological condition(s) equal or similar to disease(s) associated with the regulation of PPARα activity in the animal.


SEQUENCE LISTING

<110>  Takeda Chemical Industries, Ltd.

<120>  Disease Model Animal Carrying Heterologous PPAR-alpha Gene Introduced
       Thereinto And Use Thereof

<130>  3071WOOP

<150>  JP 2002-206162
<151>  2002-07-15

<160>  9

<170>  PatentIn version 3.1

<210>  1
<211>  1404
<212>  DNA
<213>  Homo sapiens

<220>
<221>  CDS
<222>  (1)..(1404)
<223>

<400>  1
atg gtg gac acg gaa agc cca ctc tgc ccc ctc tcc cca ctc gag gcc       48
Met Val Asp Thr Glu Ser Pro Leu Cys Pro Leu Ser Pro Leu Glu Ala
1               5                   10                  15
ggc gat cta gag agc ccg tta tct gaa gag ttc ctg caa gaa atg gga       96
Gly Asp Leu Glu Ser Pro Leu Ser Glu Glu Phe Leu Gln Glu Met Gly
            20                  25                  30
aac atc caa gag att tcg caa tcc atc ggc gag gat agt tct gga agc      144
Asn Ile Gln Glu Ile Ser Gln Ser Ile Gly Glu Asp Ser Ser Gly Ser
        35                  40                  45
ttt ggc ttt acg gaa tac cag tat tta gga agc tgt cct ggc tca gat      192
Phe Gly Phe Thr Glu Tyr Gln Tyr Leu Gly Ser Cys Pro Gly Ser Asp
    50                  55                  60
ggc tcg gtc atc acg gac acg ctt tca cca gct tcg agc ccc tcc tcg      240
Gly Ser Val Ile Thr Asp Thr Leu Ser Pro Ala Ser Ser Pro Ser Ser
65                  70                  75                  80
gtg act tat cct gtg gtc ccc ggc agc gtg gac gag tct ccc agt gga      288
Val Thr Tyr Pro Val Val Pro Gly Ser Val Asp Glu Ser Pro Ser Gly
                85                  90                  95
gca ttg aac atc gaa tgt aga atc tgc ggg gac aag gcc tca ggc tat      336
Ala Leu Asn Ile Glu Cys Arg Ile Cys Gly Asp Lys Ala Ser Gly Tyr
            100                 105                 110
cat tac gga gtc cac gcg tgt gaa ggc tgc aag ggc ttc ttt cgg cga      384
His Tyr Gly Val His Ala Cys Glu Gly Cys Lys Gly Phe Phe Arg Arg
        115                 120                 125
acg att cga ctc aag ctg gtg tat gac aag tgc gac cgc agc tgc aag      432
Thr Ile Arg Leu Lys Leu Val Tyr Asp Lys Cys Asp Arg Ser Cys Lys
    130                 135                 140
atc cag aaa aag aac aga aac aaa tgc cag tat tgt cga ttt cac aag      480
Ile Gln Lys Lys Asn Arg Asn Lys Cys Gln Tyr Cys Arg Phe His Lys

Lys Thr Glu Ser Asp Ala Ala Leu His Pro Leu Leu Gln Glu Ile Tyr
    450                 455                 460
agg gac atg tac                                                     1404
Arg Asp Met Tyr
465

<210>  2
<211>  468
<212>  PRT
<213>  Homo sapiens

<400>  2
Met Val Asp Thr Glu Ser Pro Leu Cys Pro Leu Ser Pro Leu Glu Ala
1               5                   10                  15
Gly Asp Leu Glu Ser Pro Leu Ser Glu Glu Phe Leu Gln Glu Met Gly
            20                  25                  30
Asn Ile Gln Glu Ile Ser Gln Ser Ile Gly Glu Asp Ser Ser Gly Ser
        35                  40                  45
Phe Gly Phe Thr Glu Tyr Gln Tyr Leu Gly Ser Cys Pro Gly Ser Asp
    50                  55                  60
Gly Ser Val Ile Thr Asp Thr Leu Ser Pro Ala Ser Ser Pro Ser Ser
65                  70                  75                  80
Val Thr Tyr Pro Val Val Pro Gly Ser Val Asp Glu Ser Pro Ser Gly
                85                  90                  95
Ala Leu Asn Ile Glu Cys Arg Ile Cys Gly Asp Lys Ala Ser Gly Tyr
            100                 105                 110
His Tyr Gly Val His Ala Cys Glu Gly Cys Lys Gly Phe Phe Arg Arg
        115                 120                 125
Thr Ile Arg Leu Lys Leu Val Tyr Asp Lys Cys Asp Arg Ser Cys Lys
    130                 135                 140
Ile Gln Lys Lys Asn Arg Asn Lys Cys Gln Tyr Cys Arg Phe His Lys
145                 150                 155                 160
Cys Leu Ser Val Gly Met Ser His Asn Ala Ile Arg Phe Gly Arg Met
                165                 170                 175
Pro Arg Ser Glu Lys Ala Lys Leu Lys Ala Glu Ile Leu Thr Cys Glu
            180                 185                 190
His Asp Ile Glu Asp Ser Glu Thr Ala Asp Leu Lys Ser Leu Ala Lys
        195                 200                 205
Arg Ile Tyr Glu Ala Tyr Leu Lys Asn Phe Asn Met Asn Lys Val Lys
    210                 215                 220
Ala Arg Val Ile Leu Ser Gly Lys Ala Ser Asn Asn Pro Pro Phe Val
225                 230                 235                 240
Ile His Asp Met Glu Thr Leu Cys Met Ala Glu Lys Thr Leu Val Ala
                245                 250                 255
Lys Leu Val Ala Asn Gly Ile Gln Asn Lys Glu Ala Glu Val Arg Ile
            260                 265                 270
Phe His Cys Cys Gln Cys Thr Ser Val Glu Thr Val Thr Glu Leu Thr
        275                 280                 285
Glu Phe Ala Lys Ala Ile Pro Gly Phe Ala Asn Leu Asp Leu Asn Asp
    290                 295                 300
Gln Val Thr Leu Leu Lys Tyr Gly Val Tyr Glu Ala Ile Phe Ala Met
305                 310                 315                 320
Leu Ser Ser Val Met Asn Lys Asp Gly Met Leu Val Ala Tyr Gly Asn
                325                 330                 335
Gly Phe Ile Thr Arg Glu Phe Leu Lys Ser Leu Arg Lys Pro Phe Cys
            340                 345                 350

Asp Ile Met Glu Pro Lys Phe Asp Phe Ala Met Lys Phe Asn Ala Leu
        355                 360                 365
Glu Leu Asp Asp Ser Asp Ile Ser Leu Phe Val Ala Ala Ile Ile Cys
    370                 375                 380
Cys Gly Asp Arg Pro Gly Leu Leu Asn Val Gly His Ile Glu Lys Met
385                 390                 395                 400
Gln Glu Gly Ile Val His Val Leu Arg Leu His Leu Gln Ser Asn His
                405                 410                 415
Pro Asp Asp Ile Phe Leu Phe Pro Lys Leu Leu Gln Lys Met Ala Asp
            420                 425                 430
Leu Arg Gln Leu Val Thr Glu His Ala Gln Leu Val Gln Ile Ile Lys
        435                 440                 445
Lys Thr Glu Ser Asp Ala Ala Leu His Pro Leu Leu Gln Glu Ile Tyr
    450                 455                 460
Arg Asp Met Tyr
465

<210>  3
<211>  20
<212>  DNA
<213>  Artificial Sequence

<220>
<223>  Oligonucleotide designed to act as primer for amplifying human
       SAP promoter.

<400>  3
actgagtaga agtagcagaa                                    20

<210>  4
<211>  20
<212>  DNA
<213>  Artificial Sequence

<220>
<223>  Oligonucleotide designed to act as primer for amplifying human
       SAP promoter.

<400>  4
cagcggcttg ttcatattcc                                    20

<210>  5
<211>  29
<212>  DNA
<213>  Artificial Sequence

<220>
<223>  Oligonucleotide designed to act as primer for amplifying rabbit
       beta-globin enhancer.

<400>  5
tcctaggtga gaacttcagg gtgagtttg                          29

<210>  6
<211>  29
<212>  DNA

<213>  Artificial Sequence

<220>
<223>  Oligonucleotide designed to act as primer for amplifying rabbit
       beta-globin enhancer.

<400>  6
cggtaccttt gccaaaatga tgagacagc                              29

<210>  7
<211>  15
<212>  DNA
<213>  Artificial Sequence

<220>
<223>  Oligonucleotide designed to act as primer for amplifying human
       PPAR-alpha cDNA fragment.

<400>  7
cgccagcacg gacga                                             15

<210>  8
<211>  23
<212>  DNA
<213>  Artificial Sequence

<220>
<223>  Oligonucleotide designed to act as primer for amplifying human
       PPAR-alpha cDNA fragment.

<400>  8
ttgtccccac atattcgaca ctc                                    23

<210>  9
<211>  19
<212>  DNA
<213>  Artificial Sequence

<220>
<223>  Oligonucleotide designed to act as fluorogenic probe for
       detecting human PPAR-alpha cDNA fragment amplified by PCR.

<400>  9
cccccggcag tgccctgaa                                         19

145                 150                 155                 160
tgc ctt tct gtc ggg atg tca cac aac gcg att cgt ttt gga cga atg       528
Cys Leu Ser Val Gly Met Ser His Asn Ala Ile Arg Phe Gly Arg Met
                165                 170                 175
cca aga tct gag aaa gca aaa ctg aaa gca gaa att ctt acc tgt gaa       576
Pro Arg Ser Glu Lys Ala Lys Leu Lys Ala Glu Ile Leu Thr Cys Glu
            180                 185                 190
cat gac ata gaa gat tct gaa act gca gat ctc aaa tct ctg gcc aag       624
His Asp Ile Glu Asp Ser Glu Thr Ala Asp Leu Lys Ser Leu Ala Lys
        195                 200                 205
aga atc tac gag gcc tac ttg aag aac ttc aac atg aac aag gtc aaa       672
Arg Ile Tyr Glu Ala Tyr Leu Lys Asn Phe Asn Met Asn Lys Val Lys
    210                 215                 220
gcc cgg gtc atc ctc tca gga aag gcc agt aac aat cca cct ttt gtc       720
Ala Arg Val Ile Leu Ser Gly Lys Ala Ser Asn Asn Pro Pro Phe Val
225                 230                 235                 240
ata cat gat atg gag aca ctg tgt atg gct gag aag acg ctg gtg gcc       768
Ile His Asp Met Glu Thr Leu Cys Met Ala Glu Lys Thr Leu Val Ala
                245                 250                 255
aag ctg gtg gcc aat ggc atc cag aac aag gag gcg gag gtc cgc atc       816
Lys Leu Val Ala Asn Gly Ile Gln Asn Lys Glu Ala Glu Val Arg Ile
            260                 265                 270
ttt cac tgc tgc cag tgc acg tca gtg gag acc gtc acg gag ctc acg       864
Phe His Cys Cys Gln Cys Thr Ser Val Glu Thr Val Thr Glu Leu Thr
        275                 280                 285
gaa ttc gcc aag gcc atc cca ggc ttc gca aac ttg gac ctg aac gat       912
Glu Phe Ala Lys Ala Ile Pro Gly Phe Ala Asn Leu Asp Leu Asn Asp
    290                 295                 300
caa gtg aca ttg cta aaa tac gga gtt tat gag gcc ata ttc gcc atg       960
Gln Val Thr Leu Leu Lys Tyr Gly Val Tyr Glu Ala Ile Phe Ala Met
305                 310                 315                 320
ctg tct tct gtg atg aac aaa gac ggg atg ctg gta gcg tat gga aat      1008
Leu Ser Ser Val Met Asn Lys Asp Gly Met Leu Val Ala Tyr Gly Asn
                325                 330                 335
ggg ttt ata act cgt gaa ttc cta aaa agc cta agg aaa ccg ttc tgt      1056
Gly Phe Ile Thr Arg Glu Phe Leu Lys Ser Leu Arg Lys Pro Phe Cys
            340                 345                 350
gat atc atg gaa ccc aag ttt gat ttt gcc atg aag ttc aat gca ctg      1104
Asp Ile Met Glu Pro Lys Phe Asp Phe Ala Met Lys Phe Asn Ala Leu
        355                 360                 365
gaa ctg gat gac agt gat atc tcc ctt ttt gtg gct gct atc att tgc      1152
Glu Leu Asp Asp Ser Asp Ile Ser Leu Phe Val Ala Ala Ile Ile Cys
    370                 375                 380
tgt gga gat cgt cct ggc ctt cta aac gta gga cac att gaa aaa atg      1200
Cys Gly Asp Arg Pro Gly Leu Leu Asn Val Gly His Ile Glu Lys Met
385                 390                 395                 400
cag gag ggt att gta cat gtg ctc aga ctc cac ctg cag agc aac cac      1248
Gln Glu Gly Ile Val His Val Leu Arg Leu His Leu Gln Ser Asn His
                405                 410                 415
ccg gac gat atc ttt ctc ttc cca aaa ctt ctt caa aaa atg gca gac      1296
Pro Asp Asp Ile Phe Leu Phe Pro Lys Leu Leu Gln Lys Met Ala Asp
            420                 425                 430
ctc cgg cag ctg gtg acg gag cat gcg cag ctg gtg cag atc atc aag      1344
Leu Arg Gln Leu Val Thr Glu His Ala Gln Leu Val Gln Ile Ile Lys
        435                 440                 445
aag acg gag tcg gat gct gcg ctg cac ccg cta ctg cag gag atc tac      1392